(19) Europäisches Patentamt
European Patent Office
Office européen des brevets

(11) **EP 4 254 418 A1**

(12) **EUROPEAN PATENT APPLICATION**
published in accordance with Art. 153(4) EPC

(43) Date of publication:
**04.10.2023 Bulletin 2023/40**

(21) Application number: **20962929.4**

(22) Date of filing: **27.11.2020**

(51) International Patent Classification (IPC):
**G16B 20/10** (2019.01)

(52) Cooperative Patent Classification (CPC):
**G16B 20/10**

(86) International application number:
**PCT/CN2020/132331**

(87) International publication number:
**WO 2022/110039 (02.06.2022 Gazette 2022/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **BGI Shenzhen**
**Shenzhen, Guangdong 518083 (CN)**

(72) Inventors:
• **BAI, Yong**
**Shenzhen, Guangdong 518083 (CN)**

• **HUANG, Shujia**
**Shenzhen, Guangdong 518083 (CN)**
• **GAO, Ya**
**Shenzhen, Guangdong 518083 (CN)**
• **JIN, Xin**
**Shenzhen, Guangdong 518083 (CN)**

(74) Representative: **Habermann, Hruschka & Schnabel**
**Patentanwälte**
**Montgelasstraße 2**
**81679 München (DE)**

(54) **FETAL CHROMOSOMAL ABNORMALITY DETECTION METHOD AND SYSTEM**

(57) The present invention relates to the field of biotechnology. A method and system of detecting fetal chromosomal abnormalities are disclosed. The method comprises: (1) obtaining sequencing data of cell-free nucleic acid fragments and clinical phenotypic feature data from a pregnant woman to be detected, wherein the sequencing data comprise a plurality of read segments, and the clinical phenotypic feature data of the pregnant woman form a phenotypic feature vector of the pregnant woman, (2) performing window division on at least part of a chromosome sequence of a reference genome to obtain sliding windows, counting the read segments falling within the sliding windows, and generating a sequence feature matrix of the chromosome sequence; (3) inputting the sequence feature matrix into a trained machine learning model to extract a sequence feature vector of the chromosome sequence; and (4) combining the sequence feature vector and the phenotypic feature vector of the pregnant woman to form a combined feature vector, and inputting the combined feature vector into a classification detection model to obtain the fetal chromosomal abnormality state of the pregnant woman to be detected.

FIG. 1

**Description**

**FIELD OF THE INVENTION**

The present invention relates to the field of biotechnology, and more specifically, relates to method and system of detecting fetal chromosomal abnormalities.

**BACKGROUND OF THE INVENTION**

Chromosomal aneuploidy disease refers to a kind of serious genetic diseases in which the number of individual chromosomes in the fetus increases or decreases, thereby affecting the normal gene expression. It mainly includes trisomy 21 syndrome, trisomy 18 syndrome, trisomy 13 syndrome, 5p-syndrome, etc. Chromosomal aneuploidy disease has a higher risk of death and disability, and there is no effective treatment. At present, prenatal screening and prenatal diagnosis are mainly used to reduce the birth rate of children with chromosomal aneuploidy.

The traditional chromosomal aneuploidy detection mainly includes noninvasive prenatal screening based on ultrasonic diagnostic examination or serological screening and prenatal diagnosis based on invasive sampling. The prenatal screening method based on ultrasonic diagnostic examination may be used to judge whether the fetal chromosome is abnormal by checking the thickness of the nuchal translucency (NT) of the fetus at 10-14 gestational weeks. It is generally believed that the risk of fetal chromosomal aneuploidy is higher when NT is greater than 3 mm. The prenatal screening based on serology is performed at 13-16 gestational weeks by detecting the concentrations of alpha fetoprotein (AFP) and human chorionic gonadotrophin (HCG) in the maternal serum to calculate the risk factor of fetal chromosomal abnormalities in combination with the due date and age of the pregnant woman, and the gestational week of blood sampling. The prenatal diagnostic method based on invasive sampling generally obtains fetal samples by amniocentesis, cordocentesis or direct chorionic sampling at 16-24 gestational weeks to detect whether the fetus has a chromosomal abnormality. The combined screening method based on ultrasonic diagnostic examination and serology is not to directly detect the fetal chromosomes, but to estimate the risk of fetal disease, with a detection accuracy of 50%-95% and a false positive rate of 3%-7% [1,2]. The method based on invasive sampling can directly diagnose fetal aneuploidy accurately, which is the "gold standard" for the detection and diagnosis of fetal chromosomal abnormalities. However, this method will lead to a certain of abortion rate (0.5%-2%), and the pregnant woman suffering from infectious diseases such as hepatitis B is not suitable for invasive sampling (such as amniocentesis) due to a risk of infecting the fetus. In addition, amniocentesis needs to be carried out under the guidance of B-scan ultrasonography, which takes a long time and requires high technical requirements for operators.

With the finding of cell-free DNA (cfDNA) of fetus in maternal peripheral blood, the maturity of Next Generation Sequencing (NGS) technology, the significant reduction of sequencing cost and the development of information analysis technique, Noninvasive Prenatal Testing (NIPT) based on NGS technology is becoming the most widely used prenatal screening method for fetal chromosomal aneuploidy diseases. In NIPT technology, taking use of maternal peripheral blood, sequencing cell-free DNA in maternal peripheral blood (including cell-free fetal DNA) by NGS technology, combining with bioinformatics analysis to obtain fetal genetic information, it can detect whether a fetus suffers from chromosomal abnormal diseases such as trisomy 21 syndrome (Down syndrome), trisomy 18 syndrome (Edwards syndrome) and trisomy 13 syndrome (Patau syndrome).

NIPT technology has a high sensitivity and specificity (the sensitivity of each of T21, T18 and T13 is above 99%) and a low false positive rate (< 0.1%), and it has been widely used in clinical practice [3-5]. NIPT technology can reduce the false positive rate of serological screening and avoid the risk of fetal intrauterine infection and abortion caused by invasive prenatal diagnostic operations (such as amniocentesis and chorionic villi sampling, etc.). It is a noninvasive prenatal screening technology with high safety in early and middle pregnancy.

The conventional NIPT based on NGS technology detects fetal chromosomal abnormalities by calculating the read count of sequencing and using Baseline Z-Test [6]. The principle is as follows: firstly, the maternal peripheral blood samples at 12-22 gestational weeks are taken, and the cell-free DNA in peripheral blood samples is sequenced using NGS technology, and the obtained sequencing read segments are aligned with the human reference genome sequence (and the GC content is corrected for the read count simultaneously); then the number of unique mapping reads of each chromosome is counted and its proportion to the total unique mapping read counts of chromosomes in the sample is calculated; further, the Z-score of a chromosome in the sample to be detected is obtained by subtracting the mean value of the corresponding proportion of unique mapping read counts of the chromosome in the control sample (i.e. normal sample) from the proportion of unique mapping reads of the chromosome in the sample to be detected, and then dividing by the standard deviation of the corresponding proportion of unique mapping read counts of the chromosome in the control sample; finally, the Z-score is compared with a given threshold, and it is judged as a high risk of trisomy syndrome if the Z-score is bigger than the threshold; otherwise, it is judged as a low risk. Here, the mean value of the unique mapping read counts of each chromosome in the normal samples of the control group is the Baseline Value. Thus, the more normal samples presented in the control group, the more accurate the mean value and the standard deviation of the proportion of unique mapping reads would be obtained, and thus the more accurate the Z-score would be obtained. Here the given threshold of Z-score is generally 3, which is statistically defined, *i.e.*, 99.9% deviation from normal expectation.

Different statistical hypothesis tests can be selected according to different Baseline Values. For example, correlation analysis and T-Test are adopted in reference [7], and the median of the read count of each chromosome in a fixed-size window in the sample is used as the Baseline Value, which represents the read count of this chromosome, and the median of the total read count of chromosomes in the sample is used to represent the read count of the sample; then the read count of each chromosome is divided by the read count of the sample to obtain the normalized read count of corresponding chromosome; finally, the normalized read count of each chromosome of all samples in the control group is used to calculate the confidence interval and the sample is considered to be abnormal when the score of the sample to be detected does not fall within the confidence interval. For another example, it has been proposed in reference [8] that a reference chromosome with a GC content similar to that of the chromosome of interest (such as chromosome 21) is selected in the sample of known karyotypes, and the read count of the reference chromosome is used as the baseline value for Z-test, which allow the detection accuracy of interested chromosomal abnormalities in the sample of known karyotypes reach the maximum. The reference chromosome served as the Baseline Value is the so-called Internal Chromosome. For another example, a method of Noninvasive Fetal Trisomy (NIFTY) detection has been proposed in reference [9] . In addition to comparing the read count of the chromosome with that of normal control samples, this method also considers the proportion of cell-free fetal DNA. In this method, binary hypothesis test, logarithmic likelihood ratio and FCAPS binary segmentation algorithm are used to judge detection results. NIFTY is a approach based on genome-wide. This method has been verified by a large population with high accuracy, but the process is relatively complicated. The aforementioned statistical hypothesis test (Z-Test or T-Test) method based on the read count is the key of the current NIPT analysis.

The aforementioned statistical hypothesis tests based on the read count (such as Z-Test) are currently a mainstream NIPT analysis method, but these analysis methods have obvious limitations: (1) the current NIPT analysis method may lead to the deviation of sequencing read segments distribution of individual sample, which will lead to the fluctuation of Z-score calculation in different situations, thus affecting the final result judgment and related performance indicators; (2) the current NIPT analysis method highly depends on the proportion of cell-free fetal DNA in maternal peripheral blood, and the excessively low proportion of cell-free fetal DNA (<4%) may increase the risk of false negative detection due to the large individual difference among pregnant woman; (3) the current NIPT analysis method performs well in the detection of trisomy 21 syndrome, but its accuracy in the detection of trisomy 18 syndrome and trisomy 13 syndrome is poor due to the individual difference of pregnant woman and the deviation of GC content in different chromosomes; (4) the current NIPT analysis method mainly detects the common trisomy syndrome represented by Down syndrome, and has limited clinical effect on the detection of chromosome micro-deletion and micro-duplication syndrome with also high comprehensive incidence, such as DiGeorge Syndrome, Prader-Willi Syndrome, etc. [14].

In addition, a new technology based on the machine learning model using NIPT sequencing results to detect chromosomal abnormalities has been proposed. For example, a method of assisting NIPT decisions using a Support Vector Machine (SVM) has been proposes in reference [10]. In this method, 6 different Z-score results are obtained by calculating different Baseline Values and clinical indications of the samples are also added for training the SVM model to judge chromosomal abnormalities. For another example, a Bayes method for judging chromosomal abnormalities have been designed in reference [11]. This method utilizes the prior information of cell-free fetal DNA proportion, uses Hidden Markov Model (HMM) to eliminate the interference of population level and maternal CNV, and performs GC content correction, then calculates the Bayes factor by combining with a likelihood value of Z-Test and an inferred prior value of cell-free fetal DNA proportion from the sex chromosome content. At the same time, multiple risk factors such as the age of the pregnant woman are incorporated into the prior probability to correct the Bayes factor, and the Z-score and Bayes factor are integrated to evaluate whether the chromosome is abnormal. For another example, it has been proposed in a published patent [12] that training the simple convolutional neural network model by using the NIPT sequencing results to detect chromosome copy number variation and chromosome aneuploidy abnormality. For example, it has been proposed in a published patent [13]that cell-free fetal DNA and cell-free maternal DNA may be first isolated from peripheral blood samples, and various single nucleotide variation (SNV) loci are amplified from the isolated free DNA, and the amplified products are sequenced to determine the genetic sequencing data or genetic array data of multiple SNV loci. Then, based on these genetic sequencing data or genetic array data, the artificial neural network model is trained to detect the ploidy state, tissue cancer state, or organ transplantation rejection state of the individual chromosomes.

The aforementioned methods based on machine learning model using NIPT sequencing results to detect chromosomal abnormalities also have the following limitations: most of these methods calculate the desirable features for model training based on the read count of sequencing data; most of these methods rely on the calculation of Z-score; either the calculation is too complex (e.g., reference [11]), or the model design is too simple (e.g., patent publication [12]),

or genetic sequencing data or genetic array data based on SNV loci are required (e.g., patent publication [13]), which limit clinical application prospects, model scalability and detection accuracy; and the detection accuracy needs to be improved.

## SUMMARY OF THE INVENTION

In view of the problems existing in the prior art in the detection of chromosomal abnormalities, especially aneuploidy, in order to detect chromosomal abnormalities more effectively, the invention aims at least to further improve the detection accuracy of chromosomal abnormalities based on the deep hybrid model.

Therefore, in a first aspect, the invention provides a method of detecting a fetal chromosomal abnormality, comprising:

(1) obtaining sequencing data of cell-free nucleic acid fragments and clinical phenotypic feature data from a pregnant woman to be detected, wherein the sequencing data comprise a plurality of read segments, and the clinical phenotypic feature data of the pregnant woman to be detected form a phenotypic feature vector of the pregnant woman;

(2) performing window division on at least part of a chromosome sequence of a reference genome to obtain sliding windows, counting the read segments falling within the sliding windows, and generating a sequence feature matrix of the chromosome sequence;

(3) inputting the sequence feature matrix into a trained machine learning model to extract a sequence feature vector of the chromosome sequence;

(4) combining the sequence feature vector and the phenotypic feature vector of the pregnant woman to form a combined feature vector, and inputting the combined feature vector into a classification detection model to obtain the fetal chromosomal abnormality state of the pregnant woman to be detected.

In one embodiment, in (1), the cell-free nucleic acid fragments are derived from peripheral blood, liver, and/or placenta of the pregnant woman.

In one embodiment, in (1), the cell-free nucleic acid fragments are cell-free DNA.

In one embodiment, in (1), the sequencing data are derived from ultra-low depth sequencing; preferably, the sequencing depth of the ultra-low depth sequencing is $1\times$, $0.1\times$, or $0.01\times$.

In one embodiment, in (1), the read segments are aligned to the reference genome to obtain the unique mapping reads (preferably, GC content correction is performed); preferably, the subsequent steps are carried out with the unique mapping reads (preferably, the read segments are corrected by GC content).

In one embodiment, the GC content correction is performed as follows:

a. firstly, $m$ fragments of length $\ell$ are randomly selected from a chromosome of the human reference genome;

b. the number of fragments with GC content of $i$, $N_i$, is calculated by:

$$N_i = \sum_{k=1}^{m} \mathbb{I}_i(f(k))$$

wherein $\mathbb{I}_i(f(k)) = \begin{cases} 1, & f(k) = i \\ 0, & other \end{cases}$, $f(k)$ is GC content of fragment $k$, and $i$ represents GC content ($i$ = 0%, 1%, 100%);

c. the number of sequencing read segments with GC content of $i$, $F_i$, is calculated by:

$$F_i = \sum_{k=1}^{m} c_k \cdot \mathbb{I}_i(f(k))$$

wherein $c_k \cdot \mathbb{I}_i(f(k))$ represents GC content of fragment $k$, and $F_i$ represents the number of sequencing read segments with GC content of $i$ and start site same as that of the fragment.

d. Observation-expectation ratio of GC content $\lambda_i$ is calculated by:

$$\lambda_i = \begin{cases} r \cdot \dfrac{F_i}{N_i}, & F_i > 0 \ and \ N_i > 0 \\ 1, & other \end{cases}$$

wherein $r$ is the global scaling factor, which is defined as:

$$r = \frac{\sum_i N_i}{\sum_i F_i}$$

e. the number of sequencing read segments is corrected by:

$$R_i = \frac{1}{\lambda_i}$$

wherein $R_i$ represents the expected number of sequencing read segments with a corrected GC content of $i$.

In one embodiment, in (1), the phenotypic feature data of the pregnant woman are selected from one or combination of more of: age, gestational week, height, weight, BMI, biochemical test results of prenatal examination, ultrasonic diagnosis results, and cell-free fetal DNA concentration in plasma.

In one embodiment, in (1), the phenotypic feature data of the pregnant woman are subjected to outlier processing, missing value processing and/or null value processing.

In one embodiment, the phenotypic data of the pregnant woman sample will be judged as outliers if the following records appear:

a. $x_{age} < 10$ or $x_{age} > 80$;
b. $x_{GW} < 5$ or $x_{GW} > 50$;
c. $x_{height} < 40$ or $x_{height} > 300$;
d. $x_{weight} < 10$ or $x_{weight} > 200$;

and these outliers are set as null value.

In one embodiment, the missing values and null values are padded by missForest algorithm.

In one embodiment, in (2), the chromosome is chromosome 21, chromosome 18, chromosome 13 and/or a sex chromosome.

In one embodiment, (2) comprises: (2.1) using the window with length b to overlap and slide the chromosome sequence with length L of the reference genome at step size of $t$ to obtain sliding windows, wherein b is a positive integer and b=[10000,10000000], t is any positive integer, L is a positive integer and L≥b; (2.2) counting the read segments falling within each of the sliding windows, and generating a sequence feature matrix of the chromosome sequence.

In one embodiment, in (2), the sequence feature matrix includes the number, the base quality and the mapping quality of read segments within the sliding windows.

In one embodiment, the base quality includes the mean, the standard deviation, the skewness, and/or the kurtosis of the base quality.

In one embodiment, the mapping quality includes the mean, the standard deviation, the skewness and/or the kurtosis of the mapping quality.

In one embodiment, in (2), the sequence feature matrix is:

$$\boldsymbol{X} = \left( x_{ij} \right)_{h \times w}$$

wherein $h$ represents the number of sliding windows, w represents the number of sequence features within a single sliding window, and $\boldsymbol{x}_{ij}$ represents the jth sequence eigenvalue in the ith sliding window.

In one embodiment, in (3), the sequence feature matrix is normalized.

In one embodiment, in (3), the sequence feature matrix is normalized by using formula (I):

$$Z_{i,j}^{(k)} = \frac{X_{i,j}^{(k)} - \mu_{i,j}}{\sigma_{i,j}}$$

$$(I)$$

wherein $Z_{i,j}^{(k)}$ is the normalized sequence feature matrix of sample $k$, $X_{i,j}^{(k)}$ represents the $j^{th}$ sequence eigenvalue in the $i^{th}$ sliding window of sample k, and $\mu_{i,j}$ and $\sigma_{i,j}$ represent the mean and standard deviation of the $j^{th}$ sequence eigenvalue in the $i^{th}$ sliding window of all samples, respectively.

In one embodiment, in (3), the trained machine learning model is a neural network model or an AutoEncoder model; preferably, the neural network model is a deep neural network model, and more preferably, the neural network model is a deep neural network model based on 1D convolution.

In one embodiment, the structure of the deep neural network model includes:

an input layer, for receiving the sequence feature matrix;
a pre-module, being connected with the input layer, for performing the first convolution and activation operation of the sequence feature matrix from the input layer, to obtain a feature map;
a core module, being connected with the pre-module, for further abstraction and feature extraction of the feature map from the pre-module, and strengthening the expression ability of the neural network by effectively increasing the depth of the neural network model;
a post-module, being connected with the core module, for the feature abstraction representation of the feature map from the core module;
a first global average pooling layer, being connected with the post-module, for vectorizing the feature map of the feature abstraction representation and outputting the sequence feature vector of the chromosome sequence.

In one embodiment, the pre-module includes:

(I) a 1D convolution layer;
(II) a batch normalization layer, being connected with the 1D convolution layer described in (I);
(III) a ReLU activation layer, being connected with the batch normalization layer described in (II).

In one embodiment, the core module consists of one or more residual submodules with the same structure, wherein the output of each residual module is the input of the next residual module.

In one embodiment, the residual submodule includes:

(A) a pre-submodule of the core module, each of which includes a 1D convolution layer, a Dropout layer connected with the 1D convolution layer, a batch normalization layer connected with the Dropout layer, and a ReLU activation layer connected with the batch normalization layer;
(B) a first 1D average pooling layer, being connected with the pre-submodule of the core module described in (A);
(C) a Squeeze-Excite module (SE module), and/or a Spatial Squeeze-Excite module (sSE module), being connected with the first 1D average pooling layer described in (B);
(D) a first Addition layer (Add layer), being connected with the Squeeze-Excite module and/or the Spatial Squeeze-Excite module described in (C);
(E) a second 1D average pooling layer, being connected with the ReLU activation layer in the pre-module;
(F) a second Addition layer (Add layer), being connected with the first Addition layer described in (D) and the second 1D average pooling layer described in (E).

In one embodiment, the SE module includes:

(a) a second global average pooling layer, being connected with the first 1D average pooling layer in (B) of the residual submodule;
(b) a Reshape layer, being connected with the second global average pooling layer described in (a), and the size of the output feature map of the Reshape layer is $1 \times f$, wherein $f$ is the number of 1D convolution kernels;

(c) a first fully connected layer, being connected with the Reshape layer described in (b), and the number of output neurons of the first fully connected layer is $\frac{f}{r_{SE}}$ , wherein $f$ is the number of 1D convolution kernels, and $r_{SE}$ is the decline speed of the Squeez-Excite module;

(d) a second fully connected layer, being connected with the first fully connected layer described in (c), and the number of output neurons of the second fully connected layer is $f$, wherein $f$ is the number of 1D convolution kernels;

(e) a Multiply layer, being connected with the second fully connected layer described in (d) and the first 1D average pooling layer in (B) of the residual submodule.

In one embodiment, the sSE module includes:

a. a $1 \times 1$ 1D convolution layer, being connected with the first 1D average pooling layer in (B), which uses sigmoid function as the activation function;

b. a Multiply layer, being connected with the first 1D average pooling layer in (B) and the $1 \times 1$ 1D convolution layer in a.

In one embodiment, in (4), the combined feature vector is obtained by combining the sequence feature vector and the phenotypic feature vector of the pregnant woman.

In one embodiment, in (4), the combined feature vector $x$ is normalized by:

$$x_i' = \frac{x_i - \mu_i}{\sigma_i}$$

wherein $x_i'$ is the $i^{th}$ sequence eigenvalue of the normalized combined feature vector $x$, $x_i$ is the $i^{th}$ sequence eigenvalue of the combined feature vector $x$, $\mu_i$ is the mean of the $i^{th}$ sequence eigenvalues of the combined feature vector $x$, and $\sigma_i$ is the standard deviation of the $i^{th}$ sequence eigenvalues of the combined feature vector.

In one embodiment, in (4), the classification detection model is an ensemble learning model.

In one embodiment, the ensemble learning model is an ensemble learning model based on Stacking or Majority Voting; preferably, the ensemble learning model is one or more of: support vector machine model, naive Bayes classifier, random forest classifier, XGBoost and logistic regression.

In one embodiment, the chromosomal abnormality includes at least one or more of: trisomy 21 syndrome, trisomy 18 syndrome, trisomy 13 syndrome, 5p-syndrome, chromosomal microdeletion and chromosomal microduplication.

In a second aspect, the invention provides a method of constructing a classification detection model for detecting a fetal chromosomal abnormality, comprising:

(1) obtaining sequencing data of cell-free nucleic acid fragments and clinical phenotypic feature data from a plurality of pregnant women, wherein the sequencing data comprise a plurality of read segments, and the fetal chromosomal state of each of the pregnant women is known, and the clinical phenotypic feature data of each of the pregnant women form a phenotypic feature vector of the pregnant woman;

(2) for each of the pregnant women, performing window division on at least part of a chromosome sequence of a reference genome to obtain sliding windows, counting the read segments falling within the sliding windows, and generating a sequence feature matrix of the chromosome sequence;

(3) for each of the pregnant women, constructing the training data set using the sequence feature matrix and the fetal chromosomal state, and training the machine learning model to extract a sequence feature vector of the chromosome sequence;

(4) combining the sequence feature vector and the phenotypic feature vector of each of the pregnant women to form a combined feature vector, and training the classification model with the combined feature vectors and the fetal chromosomal states of the pregnant women to obtain a trained classification detection model.

In one embodiment, the fetal chromosomal sate of each of the pregnant women is one or more of: normal diploid, chromosomal aneuploidy, partial monosomy syndrome, chromosomal microdeletion and chromosomal microduplication.

In one embodiment, the chromosomal aneuploidy includes at least one or more of: trisomy 21 syndrome, trisomy 18 syndrome and trisomy 13 syndrome.

In one embodiment, the partial monosomy syndrome includes 5p-syndrome.

In one embodiment, the number of the pregnant women is greater than 10, and the ratio of the number of fetuses with normal diploid to that of fetuses with chromosomal aneuploidy is ½ to 2.

In one embodiment, in (3), the training data set is represented as:

$$D_{seq} = \{\mathbf{Z}_{i,j}^{(k)}, y^{(k)}\}_{k=1}^{N}$$

$$y^{(k)} = \begin{cases} 1, abnormal\ fetal\ chromosomes\ of\ sample\ k \\ 0, normal\ fetal\ chromosomes\ of\ sample\ k \end{cases}$$

wherein $N$ represents the number of training samples, and $N$ is an integer $\geq 1$; $\mathbf{Z}_{i,j}^{(k)}$ is the normalized sequence feature matrix of training sample k, and $k \in [1,N]$, wherein i is an integer $\geq 1$ and j is an integer $\geq 1$.

For the same technical features as the first aspect of the invention except for the trained machine learning model, the definition in the embodiments of the first aspect of the invention also applies. In this aspect, the trained machine learning model includes an output layer. For example, the structure of the deep neural network model includes an output layer after the first global average pooling layer, and the output layer is connected with the first global average pooling layer and is a fully connected layer with a number of output neuron of 1, which is used to output the chromosomal abnormality state.

In a third aspect, the invention provides a system of detecting a fetal chromosomal abnormality, comprising:

a data acquisition module, for obtaining sequencing data of cell-free nucleic acid fragments and clinical phenotypic feature data from a pregnant woman sample to be detected, wherein the sequencing data comprise a plurality of read segments, and the clinical phenotypic feature data of the pregnant woman sample to be detected form a phenotypic feature vector of the pregnant woman;

a sequence feature matrix generation module, for performing window division on at least part of a chromosome sequence of a reference genome to obtain sliding windows, counting the read segments falling within the sliding windows, and generating a sequence feature matrix of the chromosome sequence;

a sequence feature vector extraction module, for inputting the sequence feature matrix into a trained machine learning model to extract a sequence feature vector of the chromosome sequence;

a classification detection module, for combining the sequence feature vector and the phenotypic feature vector of the pregnant woman to form a combined feature vector, and inputting the combined feature vector into a classification detection model to obtain the fetal chromosomal abnormality state of the pregnant woman to be detected.

In one embodiment, the system further includes an alignment module, for aligning the reads of the sequencing data to a reference genome to obtain the unique mapping reads.

In one embodiment, in the data acquisition module, the cell-free nucleic acid fragments are derived from the peripheral blood, liver, and/or placenta of the pregnant woman.

In one embodiment, in the data acquisition module, the cell-free nucleic acid fragments are cell-free DNA.

In one embodiment, in the data acquisition module, the sequencing data are derived from ultra-low depth sequencing; preferably, the sequencing depth of the ultra-low depth sequencing is $1\times$, $0.1\times$, or $0.01\times$.

In one embodiment, in the data acquisition module, the read segments are aligned to the reference genome to obtain the unique mapping reads (preferably, GC content correction is preformed); preferably, the subsequent steps are carried out with the unique mapping reads (preferably, the read segments are corrected by GC content).

In one embodiment, the GC content correction is performed as follows:

a. firstly, $m$ fragments of length $\ell$ are randomly selected from a chromosome of the human reference genome;
b. the number of fragments with GC content of $i$, $N_i$, is calculated by:

$$N_i = \sum_{k=1}^{m} \mathbb{I}_i(f(k))$$

$$\mathbb{I}_i(f(k)) = \begin{cases} 1, & f(k) = i \\ 0, & other \end{cases}$$

wherein , $f(k)$ is GC content of fragment $k$, and $i$ represents GC content ($i$ = 0%, 1%, 100%);

c. the number of sequencing read segments with GC content of $i$, $F_i$, is calculated by:

$$F_i = \sum_{k=1}^{m} c_k \cdot \mathbb{I}_i(f(k))$$

wherein $c_k \cdot \mathbb{I}_i(f(k))$ represents GC content of fragment $k$, and $F_i$ represents the number of sequencing read segments with GC content of $i$ and start site same as that of the fragment.

d. Observation-expectation ratio of GC content $\lambda_i$ is calculated by:

$$\lambda_i = \begin{cases} r \cdot \dfrac{F_i}{N_i}, & F_i > 0 \ and \ N_i > 0 \\ 1, & other \end{cases}$$

wherein $r$ is the global scaling factor, which is defined as:

$$r = \frac{\sum_i N_i}{\sum_i F_i}$$

e. the number of sequencing read segments is corrected by:

$$R_i = \frac{1}{\lambda_i}$$

wherein $R_i$ represents the expected number of sequencing read segments with a corrected GC content of $i$.

In one embodiment, in the data acquisition module, the phenotypic feature data of the pregnant woman are selected from one or combination of more of: age, gestational week, height, weight, BMI, biochemical test results of prenatal examination, ultrasonic diagnosis results, and cell-free fetal DNA concentration in plasma.

In one embodiment, in the data acquisition module, the phenotypic feature data of the pregnant woman are subjected to outlier processing, missing value processing and/or null value processing.

In one embodiment, in the data acquisition module, the phenotypic data of the pregnant woman sample will be judged as outliers if the following records appear:

a. $x_{age} < 10$ or $x_{age} > 80$;
b. $x_{GW} < 5$ or $x_{GW} > 50$;
c. $x_{height} < 40$ or $x_{height} > 300$;
d. $x_{weight} < 10$ or $x_{weight} > 200$;

and these outliers are set as a null value.

In one embodiment, the missing values and null values are padded by missForest algorithm.

In one embodiment, in the sequence feature matrix generation module, the chromosome is chromosome 21, chromosome 18, chromosome 13 and/or a sex chromosome.

In one embodiment, the following steps are performed in the sequence feature matrix generation module: (2.1) using the window with length b to overlap and slide the chromosome sequence with length L of the reference genome at step size of $t$ to obtain sliding windows, wherein b is a positive integer and b=[10000,10000000], t is any positive integer, L is a positive integer and L≥b; (2.2) counting the read segments falling within each of the sliding windows, and generating a sequence feature matrix of the chromosome sequence.

In one embodiment, in the sequence feature matrix generation module, the sequence feature matrix includes the number, the base quality and the mapping quality of read segments within the sliding windows.

In one embodiment, the base quality includes the mean, the standard deviation, the skewness, and/or the kurtosis of the base quality.

In one embodiment, the mapping quality includes the mean, the standard deviation, the skewness and/or the kurtosis of the mapping quality.

In one embodiment, in the sequence feature matrix generation module, the sequence feature matrix is:

$$X = \left( x_{ij} \right)_{h \times w}$$

wherein $h$ represents the number of sliding windows, $w$ represents the number of sequence features within a single sliding window, and $x_{ij}$ represents the j$^{th}$ sequence eigenvalue in the i$^{th}$ sliding window.

In one embodiment, in the sequence feature vector extraction module, the sequence feature matrix is normalized.

In one embodiment, in the sequence feature vector extraction module, the sequence feature matrix is normalized by formula (I):

$$Z_{i,j}^{(k)} = \frac{X_{i,j}^{(k)} - \mu_{i,j}}{\sigma_{i,j}}$$

(I)

wherein $Z_{i,j}^{(k)}$ is the normalized sequence feature matrix of sample $k$, $X_{i,j}^{(k)}$ represents the j$^{th}$ sequence eigenvalue in the i$^{th}$ sliding window of sample $k$, and $\mu_{i,j}$ and $\sigma_{i,j}$ represent the mean and standard deviation of the j$^{th}$ sequence eigenvalue in the i$^{th}$ sliding window of all samples, respectively.

In one embodiment, in the sequence feature vector extraction module, the trained machine learning model is a neural network model or an AutoEncoder model; preferably, the neural network model is a deep neural network model, and more preferably, the neural network model is a deep neural network model based on 1D convolution.

For the deep neural network model, the definition in the embodiments of the first aspect of the invention also applies.

In one embodiment, in the classification detection module, the combined feature vector is obtained by combining the sequence feature vector and the phenotypic feature vector of the pregnant woman.

In one embodiment, in the classification detection module, the combined feature vector $x$ is normalized by:

$$x_i' = \frac{x_i - \mu_i}{\sigma_i}$$

wherein $x_i'$ is the i$^{th}$ sequence eigenvalue of the normalized combined feature vector x, $x_i$ is the i$^{th}$ sequence eigenvalue of the combined feature vector x, $\mu_i$ is the mean of the i$^{th}$ sequence eigenvalues of the combined feature vector x, and $\sigma_i$ is the standard deviation of the i$^{th}$ sequence eigenvalues of the combined feature vector.

In one embodiment, in the classification detection module, the classification detection model is an ensemble learning model.

In one embodiment, the ensemble learning model is an ensemble learning model based on Stacking or Majority Voting; preferably, the ensemble learning model is one or more of: support vector machine model, naive Bayes classifier, random forest classifier, XGBoost and logistic regression.

In a fourth aspect, the invention provides a system of constructing a classification detection model for detecting a fetal chromosomal abnormality, comprising:

a data acquisition module, for obtaining sequencing data of cell-free nucleic acid fragments and clinical phenotypic feature data from a pregnant woman, wherein the sequencing data comprise a plurality of read segments, and the fetal chromosomal state of the pregnant woman is known, and the clinical phenotypic feature data of the pregnant woman form a phenotypic feature vector of the pregnant woman;

a sequence feature matrix generation module, for performing window division on at least part of a chromosome sequence of a reference genome to obtain sliding windows, counting the read segments falling within the sliding windows, and generating a sequence feature matrix of the chromosome sequence;

a sequence feature vector extraction module, for constructing the training data set using the sequence feature matrix and the fetal chromosomal state, and training the machine learning model to extract a sequence feature vector of the chromosome sequence;

a classification detection module, for combining the sequence feature vector and the phenotypic feature vector of a pregnant woman to form a combined feature vector, and training the classification model with the combined feature vectors and the fetal chromosomal states of a plurality of pregnant women to obtain a trained classification detection model.

In one embodiment, the system further includes an alignment module, for aligning the read segments of the sequencing data to a reference genome to obtain the unique mapping reads.

For the same technical features as the third aspect of the invention except for the trained machine learning model, the definition in the embodiments of the third aspect of the invention also applies. In this aspect, the trained machine learning model includes an output layer. For example, the structure of the deep neural network model includes an output layer after the first global average pooling layer, and the output layer is connected with the first global average pooling layer and is a fully connected layer with a number of output neuron of 1, which is used to output the chromosomal abnormality state. The method and model of the invention are based on the innovative algorithm of sequencing data instead of Z-Test, and avoid the clinical problem that it is difficult to judge depending on the threshold when the result score falls in the "grey area". Moreover, as the number of samples (e.g., sequencing data of samples and the corresponding phenotype data of pregnant women) increases, the hybrid model proposed by the invention can be automatically upgraded and optimized to improve the detection accuracy.

## BRIEF DESCRIPTION OF THE DRAWINGS

FIG. 1 illustrates a flow chart of the method of detecting fetal chromosomal abnormalities based on a deep neural network hybrid model according to an embodiment of the present invention.

FIG. 2 illustrates the calculation of a feature matrix of sequencing data according to an embodiment of the present invention.

FIG. 3 illustrates the structure of a deep neural network according to an embodiment of the present invention.

FIG. 4 illustrates a Squeeze-Excite module (SE module) according to an embodiment of the present invention.

FIG. 5 illustrates a Spatial Squeeze-Excite module (sSE module) according to an embodiment of the present invention.

FIG. 6 illustrates the missing value padding of the phenotypic data set according to an embodiment of the present invention.

FIG. 7 illustrates the structure of an ensemble learning model based on Stacking according to an embodiment of the present invention.

FIG. 8 illustrates the ROC curve of the 5-fold cross-validation training results of an ensemble learning model based on Stacking according to an embodiment of the present invention.

FIG. 9 illustrates the ROC curve evaluated by the model based on the testing set according to an embodiment of the present invention.

FIG. 10 illustrates the Precision-Recall curve evaluated by the model based on the testing set according to an embodiment of the present invention.

FIG. 11 illustrates the confusion matrix diagram when the decision threshold is the default (*i.e.,* 0.5) according to an embodiment of the present invention.

FIG. 12 illustrates the function with precision and recall as thresholds according to an embodiment of the present invention.

FIG. 13 illustrates the confusion matrix diagram when the minimum recall is 0.95 (*i.e.,* limiting type II error) according to an embodiment of the present invention.

**DETAILED DESCRIPTION OF THE EMBODIMENTS**

In the present invention, the method of detecting fetal chromosomal abnormalities can be implemented by the system of detecting fetal chromosomal abnormalities; the method of constructing a classification detection model for detecting fetal chromosomal abnormalities can be implemented by the system of detection model for detecting fetal chromosomal abnormalities.

In the present invention, the data acquisition module is used for obtaining the sequencing data of cell-free nucleic acid fragments and the clinical phenotypic feature data of a pregnant woman, wherein the sequencing data comprise a plurality of read segments, the fetal chromosomal state of the pregnant woman is known (training samples) or unknown (samples to be detected), and the clinical phenotypic feature data of the pregnant woman form a phenotypic feature vector of the pregnant woman. The data acquisition module can include a data receiving module for receiving the above data. The data acquisition module can further include a sequencer, which can obtain sequencing data by inputting the cell-free nucleic acid of a pregnant woman for sequencing. Sequencing can be high throughput sequencing, and can be ultra-low depth sequencing, and the sequencing depth of the ultra-low depth sequencing is $1\times$, $0.1\times$, or $0.01\times$. The cell-free nucleic acid can be derived from the peripheral blood, liver, and/or placenta of pregnant woman. The clinical phenotypic feature of the pregnant woman and the fetal chromosomal state of the pregnant woman (training samples) can be available in the database, wherein the fetal chromosomal state of the pregnant woman can be chromosomal aneuploidy, microdeletion and/or microduplication.

In the present invention, the alignment module is used for aligning the read segments to a reference genome to obtain the unique mapping reads. Application software that aligns the sequences to a reference genome can be available from an open-source developer, for example, from some online websites, or can be self-developed.

In the present invention, the sequence feature matrix generation module is used for performing window division on at least part of a chromosome sequence of a reference genome to obtain sliding windows, counting the read segments falling within the sliding windows, and generating a sequence feature matrix of the chromosome sequence. This can be implemented by using windows with a fixed length to slide on the chromosome sequence and windows with a fixed length may be 10k, 100k, 1M, or 10M, etc. Step size can be any length and is generally set as half of the length of sliding windows for convenient calculation. The length of chromosome sequence is only required to be greater than that of the sliding window, which can be 10k, 100k, 1M, 10M, or 100M ...... till the length of an entire chromosome. Chromosome can be the target chromosome, for example, Chromosome 21 corresponding to the detection of trisomy 21 syndrome, Chromosome 18 corresponding to the detection of trisomy 18 syndrome, Chromosome 13 corresponding to the detection of trisomy 13 syndrome, Chromosomes XY corresponding to the detection of sex chromosome abnormality, and all autosomes corresponding to the detection of chromosomal microdeletion/microduplication. For each window, the parameters including the number of reads, base quality (a measure of sequencing accuracy) and mapping quality (a measure of the accuracy of aligning the read segments to the reference genome, and the higher the mapping quality, the more unique the alignment position of the read segments to the reference genome), etc. are counted, which can be done using computer software.

In the present invention, the sequence feature extraction module is used for extracting sequence features of a chromosome sequence. For the training data set, the sequence feature vector generation module uses the sequence feature matrix and the fetal chromosomal state of the pregnant woman to construct the training data set and train a machine learning model to extract the sequence feature vector of the chromosome sequence. For the testing data, the sequence feature vector generation module uses the sequence feature matrix to construct the testing data set and input into the trained machine learning model, such as the deep neural network model, to extract the sequence feature vector of the chromosome sequence.

In the present invention, for the training data set, the classification detection module, such as the training module of the ensemble learning model, is used to train a classification detection model by the combined feature vector formed by the sequence feature vector and the phenotypic feature vector of the pregnant woman as well as the fetal chromosome state to obtain the trained classification detection model.

For the testing data set, the classification detection module is used to combine the sequence feature vector with the phenotypic feature vector of the pregnant woman to form a combined feature vector as an input, and utilize the trained classification detection model to detect chromosomal abnormality state.

The present invention proposes a completely innovative method of detecting chromosomal abnormalities, such as aneuploidy, microdeletion or microduplication. Different from traditional methods, the present invention does not detect aneuploidy directly based on the number of read segments and Z-score and does not require tedious work of data preprocessing and feature extraction selection. Instead, the invention designs a machine learning model to automatically extract the sequence feature vector from the sequence feature matrix generated from the sequencing data and combine the sequence feature vector with the clinical phenotype feature of pregnant woman, and use the classification detection model for detection, so that finally obtain the prediction result of whether there is a genetic abnormality in the fetal chromosome.

In the present invention, the machine learning model is used to automatically extract the sequence feature vector from the sequencing data, which avoids the disadvantages of traditional manual extraction of NIPT whole-genome sequence features. The method of the present invention not only fully mines the sequencing data information, but also makes full use of the clinical phenotype information of pregnant woman (the phenotype data information that can be added into the model includes maternity age, gestational week, height, weight, BMI (body mass index), biochemical test results of prenatal examination, and ultrasonic diagnosis results such as NT value, etc.), and combine the extracted sequence feature vector with the phenotypic feature vector of the pregnant woman, so as to fully mine the abundant feature data information contained in the NIPT sequencing data and the clinical phenotypic result of the pregnant woman, and ensure the high reliability and validity of the detection results. The method of the present invention not only can be used to detect the common trisomy syndrome, but also can be used to detect other chromosome defects, such as chromosomal copy number variation, chromosomal microdeletion, chromosomal microduplication, etc.

In the present invention, extracting the sequence feature vector can also be carried out by using the deep neural network model based on Autoencoder network or Variational Autoencoder network, etc.

In the present invention, an ensemble learning model based on Stacking or Majority Voting is trained to detect chromosomal abnormalities, and the findings of aneuploidy by different classifiers are fully utilized, greatly improving the accuracy of finding aneuploidy.

In the present invention, the reference genome refers to the map of human genome with normal diploid chromosomes produced by, for example, the Human Genome Project, such as hg38, hg19, etc. The reference genome can be one chromosome or more chromosomes, or it can be part of a chromosome.

The present invention is further described by specific examples below. However, the present invention is not limited by the examples.

Example 1. Example of constructing a detection model.

In an exemplary embodiment, the process and steps of the exemplary model embodiment of constructing a detection model are described below.

1. Obtaining NIPT sequencing data and alignment results.

High throughput sequencing platform BGIseq500 is used to sequence training samples (SE35 is adopted, with a sequencing depth of $0.1\times$), that is, the cell-free nucleic acid fragments of a pregnant woman. The fetal chromosomal state of the pregnant woman is known. The sequencing data are aligned to a reference genome and the repeated alignment sequences are filtered to obtain the unique mapping reads.

2. Preprocessing the unique mapping reads obtained in the above step 1, and the sequence coverage depth of each coverage region of the genome being re-corrected through the relationship between GC content and sequencing depth. The specific process is as follows (see reference [15] for details):

a. firstly, $m$ fragments of length $\ell$ are randomly selected from a chromosome (such as chromosome 21) of the human reference genome.

b. the number of fragments with GC content of $i$, $N_i$, is calculated by:

$$N_i = \sum_{k=1}^{m} \mathbb{I}_i(f(k))$$

wherein $\mathbb{I}_i(f(k)) = \begin{cases} 1, & f(k) = i \\ 0, & other \end{cases}$ , $f(k)$ is GC content of fragment $k$, and i represents GC content ($i = $ 0%, 1%, ...,100%).

c. the number of unique mapping reads with GC content of $i$, $F_i$, is calculated by:

$$F_i = \sum_{k=1}^{m} c_k \cdot \mathbb{I}_i(f(k))$$

wherein $c_k \cdot \mathbb{I}_i(f(k))$ represents GC content of fragment $k$, and $F_i$ represents the number of unique mapping reads with GC content of $i$ and start site same as that of the fragment.

d. Observation-expectation ratio of GC content $\lambda_i$, is calculated by:

$$\lambda_i = \begin{cases} r \cdot \dfrac{F_i}{N_i}, & F_i > 0 \ and \ N_i > 0 \\ 1, & other \end{cases}$$

wherein r is the global scaling factor, which is defined as:

$$r = \frac{\sum_i N_i}{\sum_i F_i}$$

e. the number of unique mapping reads is corrected by:

$$R_i = \frac{1}{\lambda_i}$$

wherein $R_i$ represents the expected number of unique mapping reads with GC content of $i$ after correction.

3. Generating a sequence feature matrix.

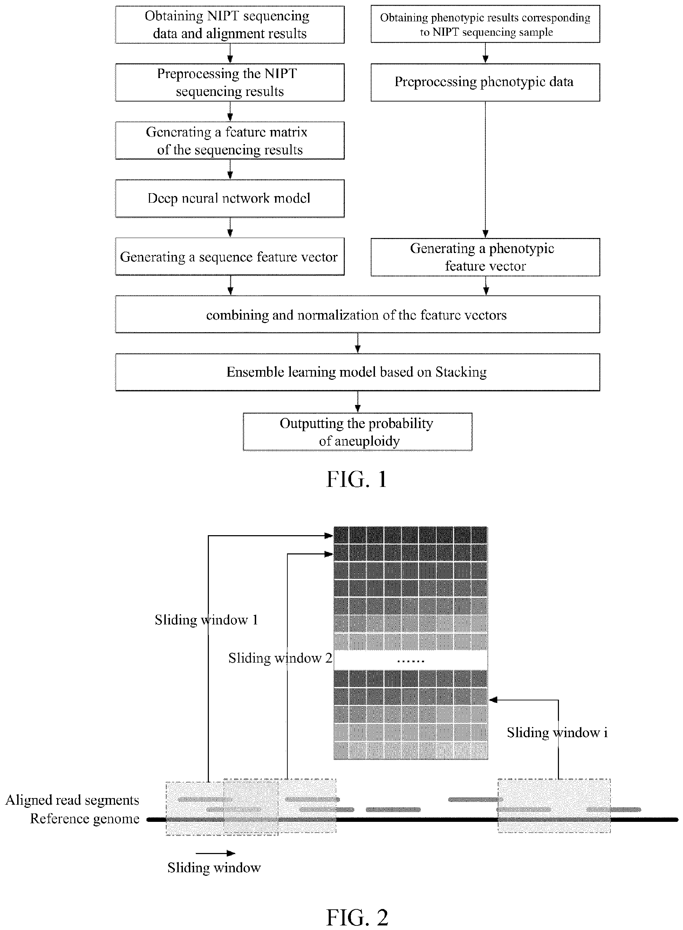

The results of the above step 2 are used to calculate the feature matrix, and the calculation process is as follows (as shown in FIG. 2):

the window with length $b$ is used to slide the target chromosome with length $L$ from start site to end site at a step size of $t$. The following features are calculated for a region with length $b$ covered by each sliding window:

a. number of GC-corrected reads in the region;
b. mean of base quality in the region;
c. standard deviation (std) of base quality in the region;
d. skewness of base quality in the region;
e. kurtosis of base quality in the region;
f. mean of mapping quality in the region;
g. standard deviation (std) of mapping quality in the region;
h. skewness of mapping quality in the region;
i. kurtosis of mapping quality in the region;

thereby, the sequence feature matrix is obtained:

$$X = \left(x_{ij}\right)_{h \times w}$$

wherein $h$ represents the number of sliding windows, for example, $h = \left\lceil \dfrac{L-b}{t} \right\rceil + 1$;
$w$ represents the number of sequence features within a single sliding window, for example, $w = 9$ (*i.e.*, 9 different features are calculated for each sliding window with length b);
and $x_{ij}$ represents the $j^{th}$ sequence eigenvalue in the $i^{th}$ sliding window.

Base quality is for quantitative description of the accuracy of sequencing results; the mean, standard deviation, skewness and kurtosis of base quality refer to the mean, standard deviation, skewness and kurtosis of all base quality in sequencing reads, respectively. Map quality refers to the reliability of the alignment of a given sequencing read segment to a reference genome sequence; the mean, standard deviation, skewness and kurtosis of map quality refer to the mean, standard deviation, skewness and kurtosis of map quality of given sequencing read segments, respectively.

4. Constructing a deep neural network model.

4.1 Constructing a data set.

The results of step 3 are used to construct the training set $D_{seq} = \{Z^{(k)}, y^{(k)}\}_{k=1}^{N}$, wherein $N$ represents the number of samples, and $N$ is an integer $\geq 1$; $Z^{(k)}$ is the normalized sequence feature matrix of sample $k$ (hereinafter referred to the normalized sequence feature matrix), and $k \in [1,N]$, being defined as: $Z_{i,j}^{(k)} = \frac{X_{i,j}^{(k)} - \mu_{i,j}}{\sigma_{i,j}}$, wherein $X_{i,j}^{(k)}$ represents the $j^{th}$ sequence feature vector in the $i^{th}$ sliding window of sample k in the training set, $\mu_{i,j}$ is the mean of the $j^{th}$ sequence feature vectors in the $i^{th}$ sliding window in the training set, $\sigma_{i,j}$ is the standard deviation of the $j^{th}$ sequence feature vectors in the $i^{th}$ sliding window in the training set, i is an integer $\geq 1$ and j is an integer $\geq 1$;

$$y^{(k)} = \begin{cases} 1, abnormal\ fetal\ chromosomes\ of\ sample\ k \\ 0, normal\ fetal\ chromosomes\ of\ sample\ k \end{cases}$$

4.2 Constructing a deep neural network model.

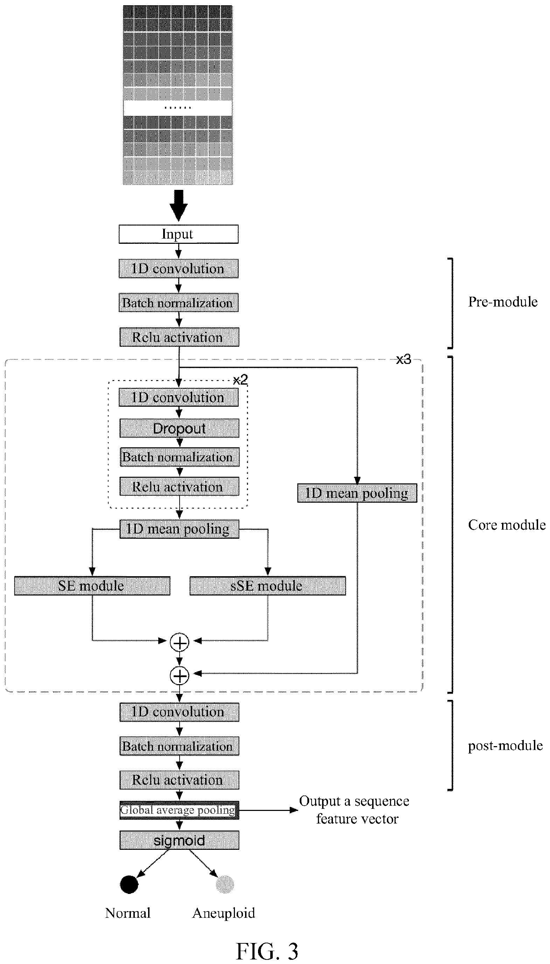

A deep neural network model is constructed, and its structure is shown in FIG. 3. All convolution layers involved in the deep neural network model are subjected to 1D convolution operations. Unless otherwise specified, the parameters of the 1D convolution kernel (i.e., 1D filter) are the same, that is, the number of 1D convolution kernels is $f$; the size of 1D convolution kernel is $k$; the step size of 1D convolution operation is $s$; the 1D convolution kernel uses L2 regularization and the regularization factor is $r_{L2}$; the initialization function of the 1D convolution kernel is g; the size of the output feature map of the 1D convolution operation is set to remain the same as that of the input feature map; the size of the pooling kernel is $p$, and the pooling step size is $p_s$.
The used Dropout Ratio of the Dropout layer involved in the deep neural network model is the same and is set as $d$.
The structure of the deep neural network model includes:

4.2.1 Input layer.

The input layer is used to receive the normalized sequence feature matrix $Z^{(k)}$ with a size of $h \times w$.

4.2.2 Pre-module.

The pre-module is connected with the input layer, and is used for performing the first convolution and activation operation of the input sequence feature matrix to obtain the abstract representation feature map. The module includes: a 1D convolution layer, a batch normalization layer connected with the 1D convolution layer, and a ReLU activation layer connected with the batch normalization layer.

4.2.3 Core module.

The core module is connected with the pre-module, and is used for further abstraction and feature extraction of the feature map, and strengthening the expression ability of the neural network by effectively increasing the depth of the neural network model. The core module consists of three repeated operations of residual modules with the same structure, wherein the output of each residual module is the input of the next residual module. Each residual module includes:

(A) a pre-submodule of the core module repeated twice, each of which has the same structure, including a 1D convolution layer, a Dropout layer connected with the 1D convolution layer, a batch normalization layer connected with the Dropout layer, and a ReLU activation layer connected with the batch normalization layer;
(B) a first 1D average pooling layer, being connected with the second pre-submodule of the core module described in (A);

(C) a Squeeze-Excite module (SE module) or a Spatial Squeeze-Excite module (sSE module), being connected with the first 1D average pooling layer described in (B);

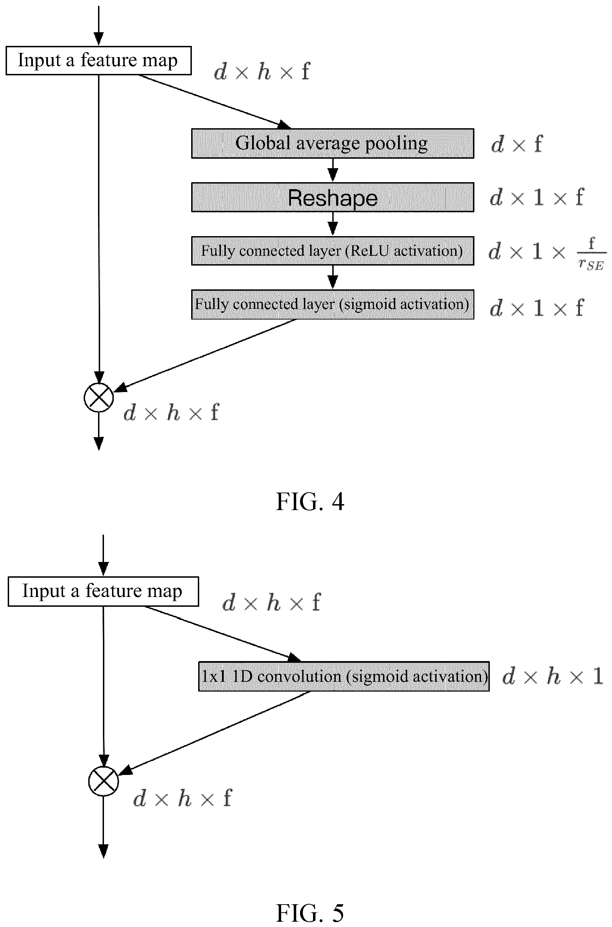

Firstly, the Reduction Ratio of SE module is set as $r_{SE}$, and as shown in FIG. 4, the structure of SE module includes (see reference [16] for details):

(a) a second global average pooling layer, being connected with the first 1D average pooling layer described in (B);
(b) a Reshape layer, being connected with the second global average pooling layer described in (a), and the size of the output feature map is $1 \times f$, wherein $f$ is the number of 1D convolution kernels;
(c) a first fully connected layer, being connected with the Reshape layer described in (b), and the number of its output neurons is $\frac{f}{r_{SE}}$, wherein $f$ is the number of 1D convolution kernels, and $r_{SE}$ is the decline speed of the SE module;
(d) a second fully connected layer, being connected with the first fully connected layer described in (c), and the number of its output neurons is $f$, wherein $f$ is the number of 1D convolution kernels;
(e) a Multiply layer, being connected with the first 1D average pooling layer described in (B) and the second fully connected layer described in (d);

as shown in FIG. 5, the structure of sSE module includes (see reference [17] for details):

a. a $1 \times 1$ 1D convolution layer, being connected with the first 1D average pooling layer described in (B), which uses sigmoid function as the activation function;
b. a Multiply layer, being connected with the first 1D average pooling layer described in (B) and the $1 \times 1$ 1D convolution layer in a;

(D) a first Addition layer (Add layer), being connected with the SE module and the sSE module described in (C);
(E) a second 1D average pooling layer, being connected with the ReLU activation layer in the pre-module described in 4.2.2;
(F) a second Addition layer (Add layer), being connected with the first Addition layer described in (D) and the second 1D average pooling layer described in (E);
(A) - (D) above are the left branch of the residual module, and (E) is the right branch of the residual module.

4.2.4 Postmodule.

The post-module has the same structure as the pre-module, and the only difference is that the number of 1D convolution kernels in the post-module is set as $n_{out}$, which is used for feature abstraction representation of the feature map from the core module before output.

4.2.5 First global average pooling layer.

The first global average pooling layer is connected with the post-module, and is used for vectorizing the feature map of the feature abstraction representation.

4.2.6 Output layer.

The output layer is connected with the first global average pooling layer, and is a fully connected layer with a number of output neuron of 1, and the activation function is sigmoid function, which is used to output the chromosomal abnormality.

5. Calculating the sequence feature vector.

The training set is used to train the deep neural network model in step 4, and the sequence feature vector of the sample is calculated using the trained deep neural network model. The process is as follows:

(1) calculating the normalized sequence feature vector of each sample according to above 4.1;

(2) inputting the normalized sequence feature matrix obtained in (1) into the deep neural network model for calculation;
(3) saving the output of the first global average pooling layer of the deep neural network model described in 4.2.5 as a generated sequence feature vector *seq* corresponding to the input sample, which is defined as:

$$seq = \left[x_1, x_2, \ldots, x_{n_{out}}\right]^T$$

wherein $n_{out}$ is the number of 1D convolution kernels defined in the post-module described in 4.2.4.

6. Obtaining the phenotypic result corresponding to the pregnant woman sample.

The phenotypic result of corresponding the pregnant woman sample is obtained, and the initial phenotypic feature vector *phe_init* is constructed, including 5 features, which is defined as:

$$phe_{init} = \left[x_{age}, x_{GW}, x_{height}, x_{weight}, x_{FF}\right]^T$$

wherein, $x_{age}$ represents the age (years) of the pregnant woman at the time of sampling, $x_{GW}$ represents the gestational week of the pregnant woman at the time of sampling, $x_{height}$ represents the height (cm) of the pregnant woman, $x_{weight}$ represents the weight (kg) of the pregnant woman, and $x_{FF}$ represents the concentration of cell-free fetal DNA in the plasma of the pregnant woman.

7. Preprocessing the phenotypic data.

Phenotypic data set of the pregnant woman is preprocessed, which includes outliers processing and missing values or null values processing.

(1) Outliers processing.

The phenotypic data of the pregnant woman sample are judged as outliers if the following records appear:

    a. $x_{age} < 10$ or $x_{age} > 80$;
    b. $x_{GW} < 5$ or $x_{GW} > 50$;
    c. $x_{height} < 40$ or $x_{height} > 300$;
    d. $x_{weight} < 10$ or $x_{weight} > 200$;

and these outliers are set as a null value.

(2) Missing values or null values processing.

The phenotypic data matrix P is constructed, which is defined as:

$$P = \left[phe_{init}^1, phe_{init}^2, \ldots phe_{init}^i, \ldots, phe_{init}^N\right]^T$$

wherein, $phe_{init}^i$ represents the phenotypic feature vector of the $i^{th}$ sample in the training set (as defined in step 6), and $N$ represents the number of samples in the training set. The samples in the training set here are consistent with those in the training set described in 4.1, so the phenotypic data matrix P is a matrix with the size of $N \times M$, wherein M is the number of phenotypic features, and here M = 5.
MissForest algorithm is used for missing value padding, which is a non-parametric missing value padding algorithm based on random forest (see reference [18] for details). Its algorithm is as follows:

---

Algorithm 1: missForest algorithm

---

Input: matrix $P$;

iterative termination condition $\gamma$;

Output: matrix $P^{imp}$ after the missing value padding;

1. $k \leftarrow$ re-establish the column index from smallest to largest according to the number of missing values in the column in the matrix $P$;

2. while not $\gamma$ do:

3. for $s$ in $k$ do:

4. extract the $s^{th}$ column $P_s$ of matrix $P$;

5. divide matrix $P$ into $\boldsymbol{y}_{obs}^{(s)}$, $\boldsymbol{y}_{mis}^{(s)}$, $\boldsymbol{X}_{obs}^{(s)}$ and $\boldsymbol{X}_{mis}^{(s)}$ according to $P_s$, wherein $\boldsymbol{y}_{obs}^{(s)}$ represents the observed values in the $P_s$ column, $\boldsymbol{y}_{mis}^{(s)}$ represents the missing values in the $P_s$ column, $\boldsymbol{X}_{obs}^{(s)}$ represents the part of the matrix $P$ with $P_s$ column and the row corresponding to $\boldsymbol{y}_{obs}^{(s)}$ removed, and $\boldsymbol{X}_{mis}^{(s)}$ represents the part of the matrix $P$ with $P_s$ column and the row corresponding to $\boldsymbol{y}_{mis}^{(s)}$ removed;

6. use the observed values to train the random forest model: $\boldsymbol{y}_{obs}^{(s)} \sim RF(\boldsymbol{X}_{obs}^{(s)})$;

7. use the trained random forest model to predict the missing values: $\boldsymbol{y}_{mis}^{(s)} = RF(\boldsymbol{X}_{mis}^{(s)})$;

8. $P^{imp} \leftarrow$ update the matrix $P$ according to the predicted values $\boldsymbol{y}_{mis}^{(s)}$;

9. end for

10. update iteration termination condition $\gamma$;

11. end while

12. return $P^{imp}$;

---

(3) Calculating BMI.

The phenotypic results after the missing value padding are used to calculate BMI, which is defined as:

$$BMI = \frac{x_{weight}}{\left(x_{height}/100\right)^2}$$

(4) Adding the result of (3) into the phenotypic feature vector after the missing value padding to obtain the final phenotypic feature vector:

$$phe = \left[x_{age}, x_{GW}, x_{height}, x_{weight}, x_{FF}, x_{BMI}\right]^{T}$$

8. Generating a combined feature vector.

The sequence feature vector described in step 5 is combined with the final feature vector described in step 7 to obtain a combined feature vector:

$$x = [seq^{T}, phe^{T}]^{T} = \left[x_{1}, x_{2}, \dots, x_{n_{out}}, x_{age}, x_{GW}, x_{height}, x_{weight}, x_{FF}, x_{BMI}\right]^{T}$$

9. Normalizing the combined feature vector.

The combined feature vector described in 8 is normalized by:

$$x_{i}' = \frac{x_{i} - \mu_{i}}{\sigma_{i}}$$

wherein $x_{i}'$ is the $i^{th}$ sequence eigenvalue of the normalized combined feature vector x, $x_{i}$ is the $i^{th}$ sequence eigenvalue of the combined feature vector x, $\mu_{i}$ is the mean of the $i^{th}$ sequence eigenvalues of the combined feature vector, and $\sigma_{i}$ is the standard deviation of the $i^{th}$ sequence eigenvalues of the combined feature vector.

10. Constructing the ensemble learning model based on Stacking.

The results described in step 9 are used to construct the training set, $D_{seq} = \{\mathbf{Z}_{i,j}^{(k)}, y^{(k)}\}_{k=1}^{N}$,

$$y^{(k)} = \begin{cases} 1, abnormal\ fetal\ chromosomes\ of\ sample\ k \\ 0, normal\ fetal\ chromosomes\ of\ sample\ k \end{cases}$$

wherein, N represents the number of training samples, and N is an integer $\geq 1$; $\mathbf{Z}_{i,j}^{(k)}$ is the normalized sequence feature matrix of training sample k, and k∈[1,N], wherein i is an integer $\geq 1$ and j is an integer>_ 1; y=0 represents normal fetal chromosome and y=1 represents abnormal fetal chromosome.
The ensemble learning algorithm based on Stacking is used to predict aneuploidy. The algorithm is as follows (see reference [19] for details):

---

Algorithm 2: Ensemble learning algorithm based on Stacking

---

Input: training set $D$;

$\quad\quad$ $M$ primary base classifiers $h_1, \ldots, h_M$;

$\quad\quad$ a secondary meta-classifier $h'$;

Output: ensemble learner $H$

1. //Step 1: train $M$ primary base classifiers

2. for $m \leftarrow 1$ to $M$ do:

3. train the base classifier $h_m$ based on the training set $D$

4. end for

5. //Step 2: construct a new data set $D'$ from the training set $D$

6. $D' = \emptyset$

7. for $i \leftarrow 1$ to $N$ do:

8. calculate vector $z_i = [h_1(x_i'), h_2(x_i'), \ldots, h_M(x_i')]^T$

9. Add vector $z_i$ into the new data set $D' = D' \cup \{(z_i, y_i)\}$

10. end for

11. //Step 3: train the secondary meta-classifier $h'$

12. train the secondary meta-classifier $h'$ based on the new data set $D'$

13. return $H(x) = h'(x) = h'(h_1(x), h_2(x), \ldots, h_M(x))$

---

Example 2. Example of detecting chromosomal abnormalities.

In an exemplary embodiment, the invention proposes a method of detecting a fetal chromosomal abnormality, which uses nucleic acid sequencing results of noninvasive prenatal testing (NIPT) and phenotypic data of a pregnant woman together to predict whether the genetic abnormality presents in fetal chromosomes. In a specific embodiment, the process and steps of the method of detecting a fetal chromosomal abnormality are shown in FIG. 1, and the specific process is described below.

1. Obtaining NIPT sequencing data and alignment results.

High throughput sequencing platform BGIseq500 is used to sequence samples to be detected (SE35 is adopted, with a sequencing depth of 0.1×). The sequencing data are aligned to a reference genome and the repeated alignment sequences are filtered to obtain the unique mapping reads.

2. Preprocessing the unique mapping reads obtained in the above step 1, and the sequence coverage depth of each coverage region of the genome being recorrected through the relationship between GC content and sequencing depth. See Example 1 for the specific process.

3. Generating a sequence feature matrix.

The results of the above step 2 are used to calculate the feature matrix, and the calculation process is as follows (as shown in FIG. 2):

the window with length b is used to slide the target chromosome with length L from start site to end site at a step size of t. The following features are calculated for a region with length b covered by each sliding window:

> a. number of GC-corrected reads in the region;
> b. mean of base quality in the region;
> c. standard deviation (std) of base quality in the region;
> d. skewness of base quality in the region;
> e. kurtosis of base quality in the region;
> f. mean of mapping quality in the region;
> g. standard deviation (std) of mapping quality in the region;
> h. skewness of mapping quality in the region;
> i. kurtosis of mapping quality in the region;

thereby, the sequence feature matrix is obtained:

$$X = \left(x_{ij}\right)_{h \times w}$$

$$h = \left\lceil \frac{L-b}{t} \right\rceil + 1 \; ;$$

wherein $h$ represents the number of sliding windows, for example,

w represents the number of sequence features within a single sliding window, for example, w = 9 (*i.e.,* 9 different features are calculated for each sliding window with length *b*);
and $x_{ij}$ represents the $j^{th}$ sequence eigenvalue in the $i^{th}$ sliding window.

Base quality is for quantitative description of the accuracy of sequencing results; the mean, standard deviation, skewness and kurtosis of base quality refer to the mean, standard deviation, skewness and kurtosis of all base quality in sequencing read segments, respectively. Map quality refers to the reliability of the alignment of a given sequence segment to a reference genome sequence; the mean, standard deviation, skewness and kurtosis of map quality refer to the mean, standard deviation, skewness and kurtosis of map quality of given sequencing read segments, respectively.

4. The trained deep neural network model in Example 1 is used to calculate the sequence feature vector of the sample, and the process is as follows:

> (1) calculating the normalized sequence feature matrix of the sample as described in 4.1 in Example 1;
> (2) inputting the normalized sequence feature matrix obtained in (1) into the deep neural network model for calculation;
> (3) saving the output of the first global average pooling layer of the deep neural network model described in 4.2.5 in Example 1 as a generated sequence feature vector *seq* corresponding to the sample, which is defined as:

$$seq = \left[x_1, x_2, \ldots, x_{n_{out}}\right]^T$$

wherein $n_{out}$ is the number of 1D convolution kernels defined in the post-module described in 4.2.4.

5. Obtaining the phenotypic result corresponding to the pregnant woman sample to be detected.

The phenotypic result corresponding to the pregnant woman samples to be detected is obtained, and the initial phenotypic feature vector $phe_{init}$ is constructed, including 5 features, which is defined as:

$$phe_{init} = \left[x_{age}, x_{GW}, x_{height}, x_{weight}, x_{FF}\right]^T$$

wherein, $x_{age}$ represents the age (years) of the pregnant woman at the time of sampling, $x_{GW}$ represents the gestational week of the pregnant woman at the time of sampling, $x_{height}$ represents the height (cm) of the pregnant woman, $x_{weight}$ represents the weight (kg) of the pregnant woman, and $x_{FF}$ represents the concentration of cell-free fetal DNA in the plasma of the pregnant woman.

6. The outliers processing for the phenotypic data

The phenotypic data of the pregnant woman sample to be detected are judged as outliers if the following records appear:

a. $x_{age} < 10$ or $x_{age} > 80$;

b. $x_{GW} < 5$ or $x_{GW} > 50$;

c. $x_{height} < 40$ or $x_{height} > 300$;

d. $x_{weight} < 10$ or $x_{weight} > 200$;

and these outliers are set as null value.

7. The sequence feature vector described in step 4 is combined with the final feature vector described in step 6 to obtain a combined feature vector:

$$x = [seq^T, phe^T]^T = \left[x_1, x_2, \ldots, x_{n_{out}}, x_{age}, x_{GW}, x_{height}, x_{weight}, x_{FF}, x_{BMI}\right]^T$$

8. Normalizing the combined feature vector.

The combined feature vector described in 7 is normalized by:

$$x_i' = \frac{x_i - \mu_i}{\sigma_i}$$

wherein $x_i'$ is the i$^{th}$ sequence eigenvalue of the normalized combined feature vector x, $x_i$ is the i$^{th}$ sequence eigenvalue of the combined feature vector x, $\mu_i$ is the mean of the i$^{th}$ sequence eigenvalues of the combined feature vector, and $\sigma_i$ is the standard deviation of the i$^{th}$ sequence eigenvalues of the combined feature vector.

9. Inputting the combined feature vector into to the ensemble learning model based on Stacking in Example 1 to obtain the fetal chromosomal state of the pregnant woman to be detected.

Example 3. Example for validation.

1. Number of Samples.

This example uses 1205 samples with "trisomy 21 (T21)" as positive samples and 1600 samples with normal chromosome (diploid) as negative samples.

Table 1 describes the number of training samples and testing samples.

| | Total number of samples | Number of samples in | Number of samples in |
|---|---|---|---|
| | (N) | training set (90%×N) | testing set (10%×N) |
| Positive samples (T21) | 1205 | 1084 | 121 |
| Negative samples (Normal) | 1600 | 1440 | 160 |

2. Preprocessing the sequencing data of all positive and negative samples according to the steps described in 2 in Example 1, wherein the number of random sampling fragments $m = 50000000$, and fragment $\ell = 180$.

3. Generating a sequence feature matrix for all positive and negative samples according to the steps described in step 3 in Example 1. The parameters are set as follows:

the length of chromosome 21: L = 46709983;

the length of sliding window: b = 1000000;

the sliding step size: $t = 500000$.

Thus, the sequence feature matrix corresponding to each sample is obtained with a size of 9 × 93, that is, $w = 9$, $h = 93$. Since the start part of chromosome 21 has no mapping sequence in the reference genome, the first 8 columns of the sequence feature matrix are filtered in this example, that is, the size of the sequence feature matrix used is actually 9 × 85.

4. Based on the result of step 3, the feature matrix of corresponded sequencing data in the training set is used to train the deep neural network model.

(1) Normalizing the feature matrix of sequencing data of the training set according to above 4.1 in Example 1, and saving the normalization model.

(2) Obtaining the input tensor with a size of $2524 \times 85 \times 9$ for training the deep neural network model according to that described in (1).

(3) Training the deep neural network model according to that described in 4.2 in Example 1 and set the parameters of the deep neural network model as follows:

the number of 1D convolution kernels: $f = 32$,

the size of 1D convolution kernel: $k = 8$,

the step size of 1D convolution operation: $s = 1$,

the 12 regularization factor of 1D convolution kernel: $r_{l2} = 0.0004$,

the initialization function of 1D convolution kernel, g, uses the "He normalization" initialization function described in reference [20],

the size of the output feature map of a 1D convolution operation is the same as that of the input feature map,

the size of pooling kernel: $p = 2$,

the step size of pooling: $p_s = 2$,

the Dropout Ratio of the Dropout layer: $r_d = 0.5$,

the decline speed of the SE module: $r_{SE} = 16$,

the number of 1D convolution kernels in the post-module $n_{out} = 8$.

This example is implemented based on the GPU versions of Tensorflow (version=1.12.2) and keras (version=2.2.4). Table 2 lists the operations of each layer, the size of the output feature map, and the network connections in the deep neural network model based on the parameters described.

| NO. | Module | Network layer | Size of output | Connection NO. |
|---|---|---|---|---|
| 1 | Input layer | Input | (None, 85, 9) | |
| 2 | | Conv1D | (None, 85, 32) | 1 |
| 3 | Pre-module | Batch Normalization | (None, 85, 32) | 2 |
| 4 | | ReLU | (None, 85, 32) | 3 |

(continued)

| NO. | Module | | | | Network layer | Size of output | Connection NO. |
|---|---|---|---|---|---|---|---|
| 5 | Core module | First residual module | Left branch of residual module | First sub mod ule | Conv1D | (None, 85, 32) | 4 |
| 6 | | | | | Dropout | (None, 85, 32) | 5 |
| 7 | | | | | Batch Normalization | (None, 85, 32) | 6 |
| 8 | | | | | ReLU | (None, 85, 32) | 7 |
| 9 | | | | Seco nd sub mod ule | Conv1D | (None, 85, 32) | 8 |
| 10 | | | | | Dropout | (None, 85, 32) | 9 |
| 11 | | | | | Batch Normalization | (None, 85, 32) | 10 |
| 12 | | | | | ReLU | (None, 85, 32) | 11 |
| 13 | | | | Aver age pooli ng layer | Average Pooling | (None, 42, 32) | 12 |
| 14 | | | | SE mod ule | Global Average Pooling | (None, 32) | 13 |
| 15 | | | | | Reshape | (None, 1, 32) | 14 |
| 16 | | | | | FC (Activation=ReLU) | (None, 1, 2) | 15 |
| 17 | | | | | FC (Activation=sigmoid) | (None, 1, 32) | 16 |
| 18 | | | | | Multiply | (None, 42, 32) | 12 and 17 |
| 19 | | | | sSE mod ule | Conv1D (Activation=sigmoid) | (None, 42, 1) | 12 |
| 20 | | | | | Multiply | (None, 42, 32) | 12 and 19 |
| 21 | | | | Add | Add | (None, 42, 32) | 18 and 20 |
| 22 | | | Right branch of residual module | Aver age pooli ng layer | Average Pooling | (None, 42, 32) | 3 |
| 23 | | | Add branches | | Add | (None, 42, 32) | 21 and 22 |

(continued)

| NO. | Module | | | | Network layer | Size of output | Connection NO. |
|---|---|---|---|---|---|---|---|
| 24 | | | | First sub mod ule | Conv1D | (None, 42, 32) | 23 |
| 25 | | | | | Dropout | (None, 42, 32) | 24 |
| 26 | | | | | Batch Normalization | (None, 42, 32) | 25 |
| 27 | | | | | ReLU | (None, 42, 32) | 26 |
| 28 | | | | Seco nd sub mod ule | Conv1D | (None, 42, 32) | 27 |
| 29 | | | | | Dropout | (None, 42, 32) | 28 |
| 30 | | | | | Batch Normalization | (None, 42, 32) | 29 |
| 31 | | | | | ReLU | (None, 42, 32) | 30 |
| 32 | | Second residual module | Left branch of residual module | Aver age pooli ng layer | Average Pooling | (None, 21, 32) | 31 |
| 33 | | | | SE mod ule | Global Average Pooling | (None, 32) | 32 |
| 34 | | | | | Reshape | (None, 1, 32) | 33 |
| 35 | | | | | FC (Activation=ReLU) | (None, 1, 2) | 34 |
| 36 | | | | | FC (Activation=sigmoid ) | (None, 1, 32) | 35 |
| 37 | | | | | Multiply | (None, 21, 32) | 32 and 36 |
| 38 | | | | sSE mod ule | Conv1D (Activation=sigmoid) | (None, 21, 1) | 32 |
| 39 | | | | | Multiply | (None, 21, 32) | 32 and 38 |
| 40 | | | | Add | Add | (None, 21, 32) | 37 and 39 |
| 41 | | | Right branch of residual module | Aver age pooli ng layer | Average Pooling | (None, 21, 32) | 23 |
| 42 | | | Add branches | | Add | (None, 21, 32) | 40 and 41 |

(continued)

| NO. | Module | | | | Network layer | Size of output | Connection NO. |
|---|---|---|---|---|---|---|---|
| 43 | | Third residual module | Left branch of residual module | First sub mod ule | Conv1D | (None, 21, 32) | 42 |
| 44 | | | | | Dropout | (None, 21, 32) | 43 |
| 45 | | | | | Batch Normalization | (None, 21, 32) | 44 |
| 46 | | | | | ReLU | (None, 21, 32) | 45 |
| 47 | | | | Seco nd sub mod ule | Conv1D | (None, 21, 32) | 46 |
| 48 | | | | | Dropout | (None, 21, 32) | 47 |
| 49 | | | | | Batch Normalization | (None, 21, 32) | 48 |
| 50 | | | | | ReLU | (None, 21, 32) | 49 |
| 51 | | | | Aver age pooli ng layer | AveragePooling | (None, 10, 32) | 50 |
| 52 | | | | SE mod ule | GlobalAveragePooling | (None, 32) | 51 |
| 53 | | | | | Reshape | (None, 1, 32) | 52 |
| 54 | | | | | FC (Activation=ReLU) | (None, 1, 2) | 53 |
| 55 | | | | | FC (Activation=sigmoid) | (None, 1, 32) | 54 |
| 56 | | | | | Multiply | (None, 10, 32) | 51 and 55 |
| 57 | | | | sSE mod ule | Conv1D | (None, 10, 1) | 51 |
| 58 | | | | | Multiply | (None, 10, 32) | 51 and 57 |
| 59 | | | | Add | Add | (None, 10, 32) | 56 and 58 |
| 60 | | | Right branch of residual module | Aver age pooli ng layer | Average Pooling | (None, 10, 32) | 42 |
| 61 | | | Add branches | | Add | (None, 10, 32) | 59 and 60 |

(continued)

| NO. | Module | Network layer | Size of output | Connection NO. |
|---|---|---|---|---|
| 62 | | Conv1D | (None, 10, 8) | 61 |
| 63 | Post-module | Batch Normalization | (None, 10, 8) | 62 |
| 64 | | ReLU | (None, 10, 8) | 63 |
| 65 | Global average pooling layer | Global Average Pooling | (None, 8) | 64 |
| 66 | Output layer | FC (Activation=sigmoid) | (None, 1) | 65 |

(4) 80% of the samples in the training set are used for training the deep neural network, and 20% are used for verification to calculate the accuracy.

(5) The training for the deep neural network is set as follows: the iterations epochs = 100 and the size of sample batch mini_batch = 64. Adam algorithm is used as optimization algorithm of gradient descent (parameter $\beta_1$ = 0.9, $\beta_2$ = 0.999), and the initial learning rate is set as 0.01. In the training process, if the accuracy rate does not improve after successive 2 iterations, the learning rate will be reduced by 2 times (*i.e.*, multiplied by 0.5); if the accuracy rate does not improve after 10 successive iterations, the training stops.

(6) Class weight factor is introduced to the training process of the deep neural network model (using compute_class_weight() function in machine learning library scikit-learn (version=0.22.2) to calculate the class weight, and assigning the class weight to the samples of the corresponding class).

(7) The trained deep neural network model is saved.

5. Calculating the sequence feature vector according to step 5 in above Example 1:

(1) Calculating the sequence feature matrix for all samples in the whole data set (including the training set and the testing set) according to step 3 in above Example 1;

(2) Normalizing the sequence feature matrix obtained in above (1) using the obtained sequence normalization model according to that described in 4.1;

(3) Inputting the result of above (2) into the deep neural network model obtained in 4 and modifying the output layer of the model to the global average pooling layer (*i.e.,* the 65th layer in Table 2);

(4) Obtaining the sequence feature vectors of all samples in the whole data set (including the training set and testing set) according to the process of (3).

6. According to step 7 in above Example 1, the phenotypic features of all samples in the whole data set (including the training set and testing set) are obtained and the outliers of the phenotypic features are processed.

7. According to step 7 in above Example 1, the phenotypic features in the training set are subjected to the missing value padding, and the padding model of the missing values is saved.

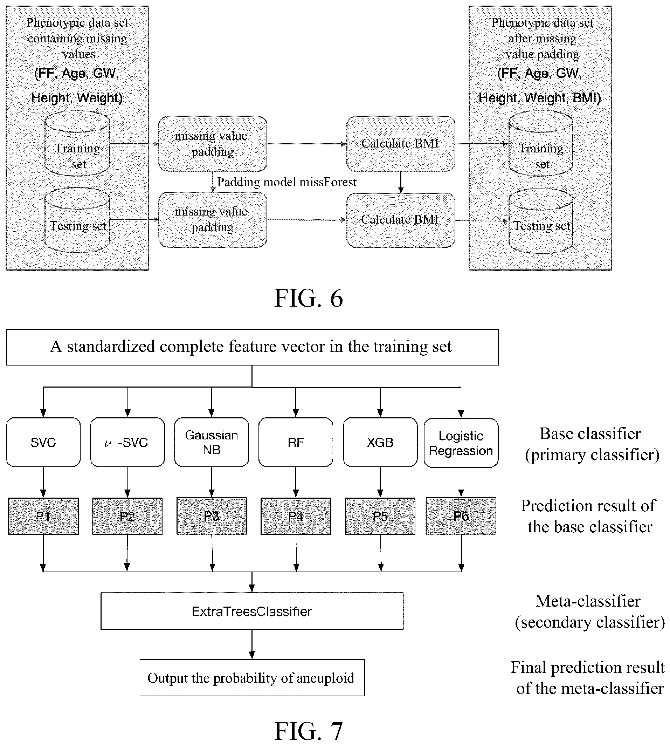

8. According to step 7 in above Example 1, BMI is calculated for the phenotypic features in the training set after the missing value processing, as shown in FIG. 6.

9. According to step 8 in above Example 1, the sequence feature vector in the training set is combined with the phenotypic feature vector of the corresponding sample to obtain a combined feature vector.

10. According to step 9 in above Example 1, the combined feature vector of each sample in the training set is normalized to obtain the normalized feature vector and the normalization model of the combined feature vector is saved.

11. According to the process of above steps 7-10, the saved padding model of the missing values is used for the missing value padding of the phenotypic features of each sample in the testing set, and the sequence feature vector of the testing set is then combined with the phenotypic feature vector of the corresponding sample to obtain the combined feature vector of the testing set, and then the saved normalization model of the combined feature vector is used to normalize the combined feature vector in the testing set.

12. The normalized feature vector of the training set obtained in above step 10 is used to train the ensemble learning model based on Stacking, as shown in FIG. 7. This example is implemented based on the scikit-learn (version=0.22.2) machine learning library, wherein class weight factor is introduced into each base classifier model and the final meta-classifier model, and a parameter is set as default value if not otherwise specified.

(1) According to step 10 in above Example 1, the base classifier used in the example includes:

- SVC, with the parameters of C=0.5, kernel="rbf"

- v-svc, with the parameters of v=0.25, kernel="rbf"

- GaussianNB (Gaussian Naive Bayes model)

- RandomForestClassifier (Random forest classifier), with the parameters of n_estimators=100, criterion="gini", max_depth=5, min_samples_leaf=1 and min_samples_split=2

- XGBClassifier (XGBoosting classifier), with the parameters of n_estimators=100, min_child_weight=1, gamma=0.1, colsample_bytree=0.8, subsample=0.7, reg_alpha=0.01, max_depth=5 and learning_rate = 0.05,

- LogisticRegression, with the parameter C=0.5

(2) According to step 10 in Example 1, the final meta-classifier is ExtraTreesClassifier (extremely randomized tree classifier). The parameters involved in this classifier are set as n_estimators=110, max_depth=6, min_samples_split=3 and min_samples-leaf=1, respectively.

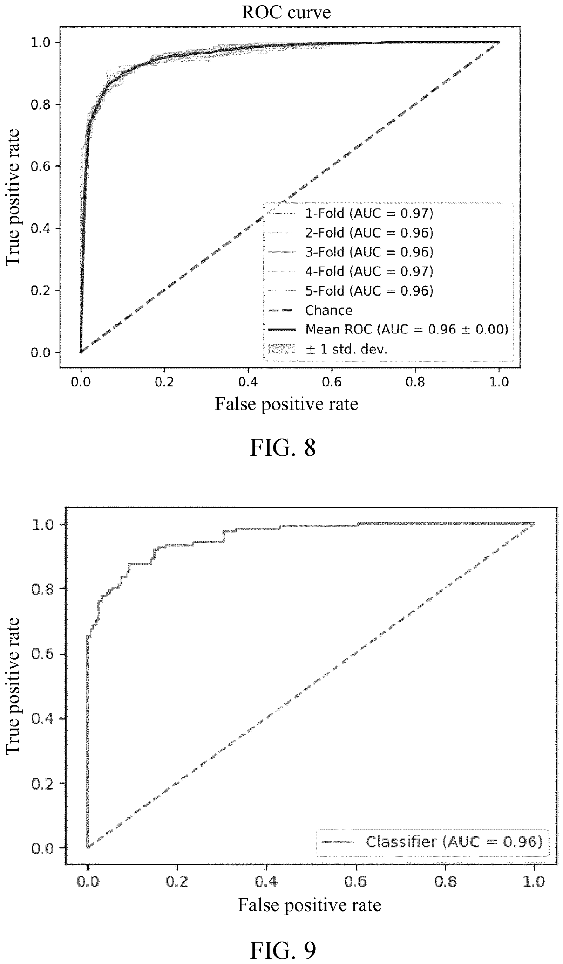

(3) 5-fold cross-validation training is performed for the ensemble learning model based on Stacking and the result is shown in FIG. 8. It shows the average AUC obtained by using the 5-fold cross-validation to train the model is 0.96.

13. The trained ensemble learning model based on Stacking described in step 12 is verified using the testing set.

(1) ROC curve of testing result is shown in FIG. 9, wherein AUC=0.96;

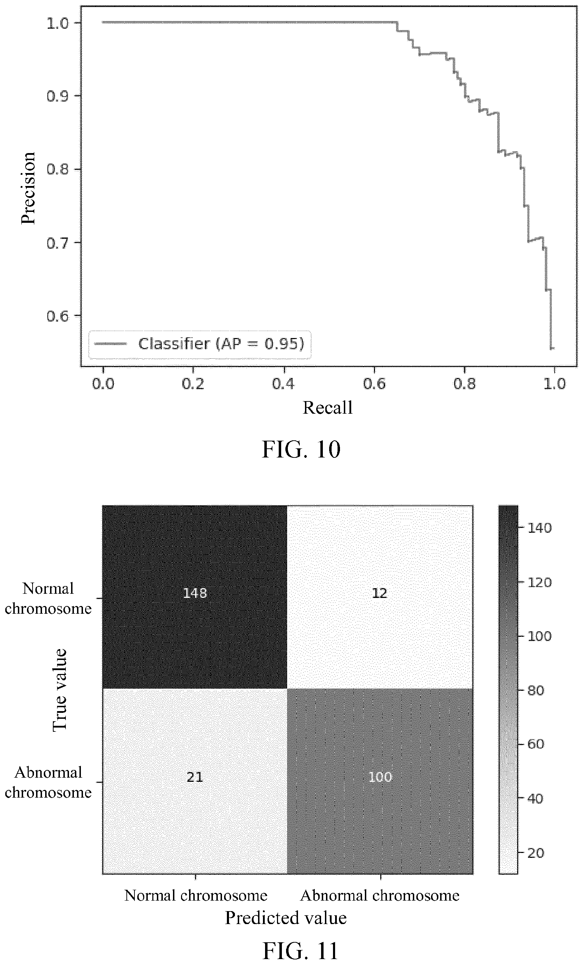

(2) Precision-Recall curve of testing result is shown in FIG. 10, wherein AP=0.95.

(3) With the default decision threshold (*i.e.,* 0.5), the confusion matrix is shown in FIG. 11; the recall and the precision are 0.83 and 0.89, respectively.

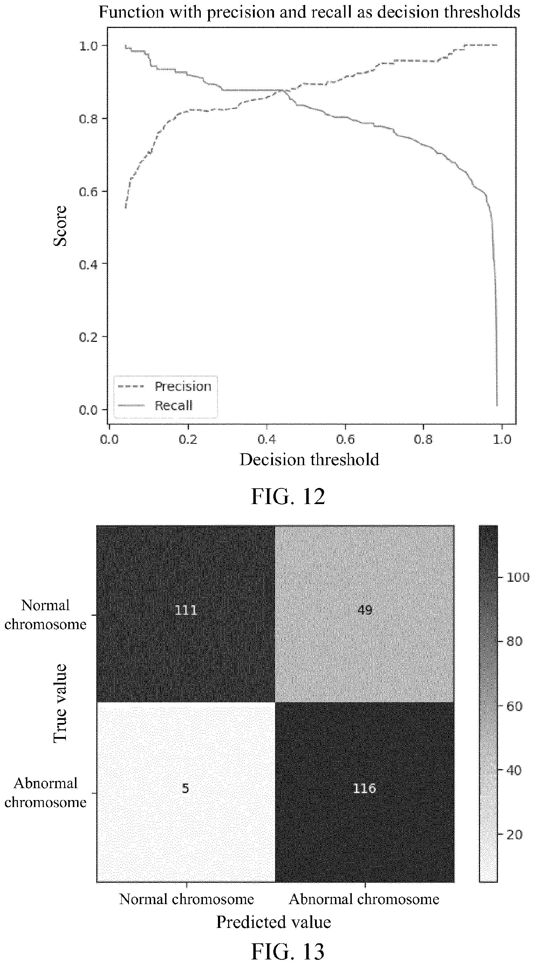

(4) The function with the precision and the recall as decision threshold is shown in FIG. 12.

(5) The minimum recall is set as 0.95 (*i.e.,* limiting type II error). The results obtained are shown in FIG. 12; the recall and the precision are 0.96 and 0.70, respectively.

The invention proposes using a machine learning model (such as a deep neural network) to extract the sequence feature vector of NIPT sequencing data, and then combining the sequence feature vector (the features including but not limited to the read count, base quality and mapping quality) with the phenotypic feature vector of the pregnant woman (the phenotypic features of pregnant woman including but not limited to maternity age, gestational week, height, weight, BMI, biochemical test results of the prenatal examination, and ultrasonic diagnosis results such as NT value, etc.) to form a vector combination, and then using a classification model, such as an ensemble learning model based on Stacking, to obtain the final predictive aneuploidy. In the present invention, extracting the sequence feature vector is not limited to the method used herein but also can be used including but not limited to an Autoencoder network or a Variational Autoencoder network. The model structure proposed by the invention is a hybrid model, that is, the model comprises 2 stages. In the first stage, a machine learning model (such as a deep neural network) is used to calculate the sequence feature vector. In the second phase, a classification model (such as an ensemble learning model based on Stacking) is used to predict aneuploidy by using the combination of sequence feature vector and phenotypic feature vector. Other ensemble learning models, such as a model based on Majority Voting, can also be used.

Compared with other convolutional neural networks, the verified advanced deep neural network model used in the examples of the invention has the following features on network design and architecture: the deep neural network model used in the examples of the invention is a deep neural network model based on 1D convolutional model; the deep neural network model used in the examples of the invention is a network model based on residual network; the SE module of Squeeze-Excite network is introduced into the deep neural network model used in the examples of the invention. Based on these designs, the neural network model used in the examples of the invention has more layers (see Example 3), and effectively reduces the risk of gradient disappearance and overfitting in the process of training model, and improves the stability, and thus effectively improves the accuracy of model prediction result.

The invention can be implemented as a computer-readable storage medium, on which a computer program is stored, and the steps to implement the method of the invention are executed when the computer program is executed by a processor. In one example, the computer program is distributed over several computer devices or processors coupled by network, so that the computer program is stored, accessed, and executed in a distributed manner by one or more computer devices or processors. A single step/operation, or two or more steps/operations, can be executed by a single computer device or processor or by two or more computer devices or processors. One or more steps/operations can be executed by one or more computer devices or processors, and one or more other steps/operations can be executed by one or more other computer devices or processors. One or more computer devices or processors can execute a single step/operation, or two or more steps/operations.

Those skilled in the art will appreciate that the division and order of the steps in the method of detecting fetal chromosomal abnormalities of the present invention are illustrative only and are not limiting, and deletions, additions, replacements, modifications and variations can be conducted by those skilled in the art without departing from the spirit or scope of the present invention as set forth in the appended claims and equivalent technical solutions thereof. The technical features of the embodiments of the invention can be combined optionally and not all possible combinations of the technical features in the above embodiments are described for making the description concise. However, it should be considered fall within the scope of the specification, if there is no conflict among the combinations of these technical features.

Although the invention has been described with reference to exemplary embodiments, it should be understood that the invention is not limited to the constructions and methods of the above embodiments. Instead, the invention is intended to cover various modifications and equivalent configurations. In addition, although various elements and method steps disclosed in the present invention are shown in various exemplary combinations and constructions, other combinations comprising more or less elements or methods also fall within the scope of the present invention.

References:

[1] Evans, Mark I., Stephanie Andriole, and Shara M. Evans. "Genetics: update on prenatal screening and diagnosis." Obstetrics and Gynecology Clinics 42.2 (2015): 193-208.

[2] Norwitz, Errol R., and Brynn Levy. "Noninvasive prenatal testing: the future is now." Reviews in obstetrics and gynecology 6.2 (2013): 48.

[3] Norton, Mary E., et al. "Cell-free DNA analysis for noninvasive examination of trisomy." New England Journal of Medicine 372.17 (2015): 1589-1597.

[4] Langlois, Sylvie, et al. "Current status in non-invasive prenatal detection of Down syndrome, trisomy 18, and trisomy 13 using cell-free DNA in maternal plasma." Journal of Obstetrics and Gynaecology Canada 35.2 (2013): 177-181.

[5] Allyse, Megan, et al. "Non-invasive prenatal testing: a review of international implementation and challenges." International journal of women's health 7 (2015): 113.

[6] Chiu, Rossa WK, et al. "Noninvasive prenatal diagnosis of fetal chromosomal aneuploidy by massively parallel genomic sequencing of DNA in maternal plasma." Proceedings of the National Academy of Sciences 105.51 (2008): 20458-20463.

[7] Fan, H. Christina, et al. "Noninvasive diagnosis of fetal aneuploidy by shotgun sequencing DNA from maternal blood." Proceedings of the National Academy of Sciences 105.42 (2008): 16266-16271.

[8] Lau, Tze Kin, et al. "Noninvasive prenatal diagnosis of common fetal chromosomal aneuploidies by maternal plasma DNA sequencing." The Journal of Maternal-Fetal & Neonatal Medicine 25.8 (2012): 1370-1374.

[9] Jiang, Fuman, et al. "Noninvasive Fetal Trisomy (NIFTY) test: an advanced noninvasive prenatal diagnosis methodology for fetal autosomal and sex chromosomal aneuploidies." BMC medical genomics 5.1 (2012): 57.

[10] Yang, Jianfeng, Xiaofan Ding, and Weidong Zhu. "Improving the calling of non-invasive prenatal testing on 13-/18-/21-trisomy by support vector machine discrimination." BioRxiv (2017): 216689.

[11] Xu, Hanli, et al. "Informative priors on fetal fraction increase power of the noninvasive prenatal screen." Genetics in Medicine 20.8 (2018): 817-824.

[12] Ehrich, Mathias, et al. "Deep learning-based methods, devices, and systems for prenatal testing", Publication number: WO2019191319A1, Filing Date: 27 March 2019.

[13] Egilsson, Agust, et al. "Methods and systems for calling ploidy status using a neural network". Publication number: WO2020018522A1, Filing date: 16 July 2019.

[14] Petersen, Andrea K., et al. "Positive predictive value estimates for cell-free noninvasive prenatal screening from data of a large referral genetic diagnostic laboratory." American journal of obstetrics and gynecology 217.6 (2017): 691-e1.

[15] Benjamini, Yuval, and Terence P. Speed. "Summarizing and correcting the GC content bias in high-throughput sequencing." Nucleic acids research 40.10 (2012): e72-e72.

[16] Hu, Jie, Li Shen, and Gang Sun. "Squeeze-and-excitation networks." Proceedings of the IEEE conference on computer vision and pattern recognition. 2018.

[17] Roy, Abhijit Guha, Nassir Navab, and Christian Wachinger. "Concurrent spatial and channel 'squeeze & excitation'in fully convolutional networks." International Conference on Medical Image Computing and Computer-Assisted Intervention. Springer, Cham, 2018.

[18] Stekhoven, Daniel J., and Peter Bühlmann. "MissForest-non-parametric missing value imputation for mixed-type data." Bioinformatics 28.1 (2012): 112-118.

[19] Tang, J., S. Alelyani, and H. Liu. "Data Classification: Algorithms and Applications." Data Mining and Knowledge Discovery Series, CRC Press (2015): pp. 498-500.

[20] He, Kaiming, et al. "Delving deep into rectifiers: Surpassing human-level performance on imagenet classification." Proceedings of the IEEE international conference on computer vision. 2015.

## Claims

1. A method of detecting a fetal chromosomal abnormality, comprising:

   (1) obtaining sequencing data of cell-free nucleic acid fragments and clinical phenotypic feature data from a pregnant woman to be detected, wherein the sequencing data comprise a plurality of read segments, and the clinical phenotypic feature data of the pregnant woman to be detected form a phenotypic feature vector of the pregnant woman;
   (2) performing window division on at least part of a chromosome sequence of a reference genome to obtain sliding windows, counting the read segments falling within the sliding windows, and generating a sequence feature matrix of the chromosome sequence;
   (3) inputting the sequence feature matrix into a trained machine learning model to extract a sequence feature vector of the chromosome sequence;
   (4) combining the sequence feature vector and the phenotypic feature vector of the pregnant woman to form a combined feature vector, and inputting the combined feature vector into a classification detection model to obtain the fetal chromosomal abnormality state of the pregnant woman to be detected.

2. The method of Claim 1, in (1), the cell-free nucleic acid fragments are derived from the peripheral blood, liver, and/or placenta of the pregnant woman.

3. The method of Claim 1 or 2, in (1), the cell-free nucleic acid fragments are cell-free DNA.

4. The method of any one of Claims 1-3, in (1), the sequencing data are derived from ultra-low depth sequencing; preferably, the sequencing depth of the ultra-low depth sequencing is $1\times$, $0.1\times$, or $0.01\times$.

5. The method of any one of Claims 1-4, in (1), the read segments are aligned to the reference genome to obtain the unique mapping reads (preferably, GC content correction is performed); preferably, the subsequent steps are carried out with the unique mapping reads (preferably, the read segments are corrected by GC content).

6. The method of Claim 5, the GC content correction is performed as follows:

   a. firstly, $m$ fragments of length $\ell$ are randomly selected from a chromosome of the human reference genome;
   b. the number of fragments with GC content of $i$, $N_i$, is calculated by:

$$N_i = \sum_{k=1}^{m} \mathbb{I}_i(f(k))$$

wherein $\mathbb{I}_i(f(k)) = \begin{cases} 1, & f(k) = i \\ 0, & other \end{cases}$ , $f(k)$ is GC content of fragment $k$, and $i$ represents GC content ($i = 0\%, 1\%, ...,100\%$);

c. the number of sequencing read segments with GC content of $i$, $F_i$, is calculated by:

$$F_i = \sum_{k=1}^{m} c_k \cdot \mathbb{I}_i(f(k))$$

wherein $c_k \cdot \mathbb{I}_i(f(k))$ represents GC content of fragment $k$, and $F_i$ represents the number of sequencing read segments with GC content of i and start site same as that of the fragment.

d. Observation-expectation ratio of GC content $\lambda_i$ is calculated by:

$$\lambda_i = \begin{cases} r \cdot \dfrac{F_i}{N_i}, & F_i > 0 \ and \ N_i > 0 \\ 1, & other \end{cases}$$

wherein r is the global scaling factor, which is defined as:

$$r = \frac{\sum_i N_i}{\sum_i F_i}$$

e. the number of sequencing read segments is corrected by:

$$R_i = \frac{1}{\lambda_i}$$

wherein $R_i$ represents the expected number of sequencing read segments with a correcte GC content of $i$.

7. The method of any one of Claims 1-6, in (1), the phenotypic feature data of the pregnant woman are selected from one or combination of more of: age, gestational week, height, weight, BMI, biochemical test results of prenatal examination, ultrasonic diagnosis results, and cell-free fetal DNA concentration in plasma.

8. The method of any one of Claims 1-7, in (1), the phenotypic feature data of the pregnant woman are subjected to outlier processing, missing value processing and/or null value processing.

9. The method of Claim 8, in (1), the phenotypic data of the pregnant woman sample will be judged as outliers if the following records appear:

a. $x_{age} < 10$ or $x_{age} > 80$;
b. $x_{GW} < 5$ or $x_{GW} > 50$;
c. $x_{height} < 40$ or $x_{height} > 300$;
d. $x_{weight} < 10$ or $x_{weight} > 200$;

and these outliers are set as null values.

**10.** The method of Claim 8 or 9, the missing values and null values are padded by missForest algorithm.

**11.** The method of any one of Claims 1-10, in (2), the chromosome is chromosome 21, chromosome 18, chromosome 13 and/or a sex chromosome.

**12.** The method of any one of Claims 1-11, (2) comprises: (2.1) using the window with length b to overlap and slide the chromosome sequence with length L of the reference genome at step size of t to obtain sliding windows, wherein b is a positive integer and b=[10000,10000000], t is any positive integer, L is a positive integer and L≥b; (2.2) counting the read segments falling within each of the sliding windows, and generating a sequence feature matrix of the chromosome sequence.

**13.** The method of any one of Claims 1-12, in (2), the sequence feature matrix includes the number, the base quality and the mapping quality of read segments within the sliding windows.

**14.** The method of Claim 13, the base quality includes the mean, the standard deviation, the skewness, and/or the kurtosis of the base quality.

**15.** The method of Claim 13, the mapping quality includes the mean, the standard deviation, the skewness and/or the kurtosis of the mapping quality.

**16.** The method of any one of Claims 1-15, in (2), the sequence feature matrix is:

$$X = \left(x_{ij}\right)_{h \times w}$$

wherein $h$ represents the number of sliding windows, $w$ represents the number of sequence features within a single sliding window, and $x_{ij}$ represents the $j^{th}$ sequence eigenvalue in the $i^{th}$ sliding window.

**17.** The method of any one of Claims 1-16, in (3), the sequence feature matrix is normalized.

**18.** The method of Claim 17, in (3), the sequence feature matrix is normalized by using formula (I):

$$Z_{i,j}^{(k)} = \frac{X_{i,j}^{(k)} - \mu_{i,j}}{\sigma_{i,j}}$$

$$(I)$$

wherein $Z_{i,j}^{(k)}$ is the normalized sequence feature matrix of sample $k$, $X_{i,j}^{(k)}$ represents the $j^{th}$ sequence eigenvalue in the $i^{th}$ sliding window of sample $k$, and $\mu_{i,j}$ and $\sigma_{i,j}$ represent the mean and standard deviation of the $j^{th}$ sequence eigenvalue in the $i^{th}$ sliding window of all samples, respectively.

**19.** The method of any one of Claims 1-18, in (3), the trained machine learning model is a neural network model or an AutoEncoder model; preferably, the neural network model is a deep neural network model, and more preferably, the neural network model is a deep neural network model based on 1D convolution.

**20.** The method of any one of Claims 1-19, the structure of the deep neural network model includes:

an input layer, for receiving the sequence feature matrix;
a pre-module, being connected with the input layer, for performing the first convolution and activation operation of the sequence feature matrix from the input layer, to obtain a feature map;
a core module, being connected with the pre-module, for further abstraction and feature extraction of the feature map from the pre-module, and strengthening the expression ability of the neural network by effectively increasing the depth of the neural network model;
a post-module, being connected with the core module, for the feature abstraction representation of the feature map from the core module;

a first global average pooling layer, being connected with the post-module, for vectorizing the feature map of the feature abstraction representation and outputting the sequence feature vector of the chromosome sequence.

21. The method of Claim 20, the pre-module includes:

(I) a 1D convolution layer;

(II) a batch normalization layer, being connected with the 1D convolution layer described in (I);

(III) ReLU activation layer, being connected with the batch normalization layer described in (II).

22. The method of Claim 20 or 21, the core module consists of one or more residual submodules with the same structure, wherein the output of each residual module is the input of the next residual module.

23. The method of any one of Claims 20-22, the residual submodule includes:

(A) a pre-submodule of the core module, each of which includes a 1D convolution layer, a Dropout layer connected with the 1D convolution layer, a batch normalization layer connected with the Dropout layer, and a ReLU activation layer connected with the batch normalization layer;

(B) a first 1D average pooling layer, being connected with the pre-submodule of the core module described in (A);

(C) a Squeeze-Excite module, and/or a Spatial Squeeze-Excite module, being connected with the first 1D average pooling layer described in (B);

(D) a first Addition layer, being connected with the Squeeze-Excite module and/or Spatial Squeeze-Excite module described in (C);

(E) a second 1D average pooling layer, being connected with the ReLU activation layer in the pre-module;

(F) a second Addition layer, being connected with the first Addition layer described in (D) and the second 1D average pooling layer described in (E).

24. The method of Claim 23, the Squeeze-Excite module includes:

(a) a second global average pooling layer, being connected with the first 1D average pooling layer in (B) of the residual submodule;

(b) a Reshape layer, being connected with the second global average pooling layer described in (a), and the size of the output feature map of the Reshape layer is $1 \times f$, wherein $f$ is the number of 1D convolution kernels;

(c) a first fully connected layer, being connected with the Reshape layer described in (b), and the number of output neurons of the first fully connected layer is $\frac{f}{r_{SE}}$, wherein $f$ is the number of 1D convolution kernels, and $r_{SE}$ is the decline speed of the Squeez-Excite module;

(d) a second fully connected layer, being connected with the first fully connected layer described in (c), and the number of output neurons of the second fully connected layer is $f$, wherein $f$ is the number of 1D convolution kernels;

(e) a Multiply layer, being connected with the second fully connected layer described in (d) and the first 1D average pooling layer in (B) of the residual submodule.

25. The method of Claim 23 or 24, the Spatial Squeeze-Excite module includes:

a. a $1 \times 1$ 1D convolution layer, being connected with the first 1D average pooling layer in (B), which uses sigmoid function as the activation function;

b. a Multiply layer, being connected with the first 1D average pooling layer in (B) and the $1 \times 1$ 1D convolution layer in a.

26. The method of any one of Claims 1-25, in (4), the combined feature vector is obtained by combining the sequence feature vector and the phenotypic feature vector of the pregnant woman.

27. The method of any one of Claims 1-26, in (4), the combined feature vector $x$ is normalized by:

$$x_i' = \frac{x_i - \mu_i}{\sigma_i}$$

wherein $x_i'$ is the i<sup>th</sup> sequence eigenvalue of the normalized combined feature vector x, $x_i$ is the i<sup>th</sup> sequence eigenvalue of the combined feature vector, $\mu_i$ is the mean of the i<sup>th</sup> sequence eigenvalues of the combined feature vector x, and $\sigma_i$ is the standard deviation of the i<sup>th</sup> sequence eigenvalues of the combined feature vector.

28. The method of any one of Claims 1-27, in (4), the classification detection model is an ensemble learning model.

29. The method of Claim 28, the ensemble learning model is an ensemble learning model based on Stacking or Majority Voting; preferably, the ensemble learning model is one or more of: support vector machine model, naive Bayes classifier, random forest classifier, XGBoost and logistic regression.

30. The method of Claim 1, the chromosomal abnormality includes at least one or more of: trisomy 21 syndrome, trisomy 18 syndrome, trisomy 13 syndrome, 5p-syndrome, chromosomal microdeletion and chromosomal microduplication.

31. A method of constructing a classification detection model for detecting a fetal chromosomal abnormality, comprising:

(1) obtaining sequencing data of cell-free nucleic acid fragments and clinical phenotypic feature data from a plurality of pregnant women, wherein the sequencing data comprise a plurality of read segments, and the fetal chromosomal state of each of the pregnant women is known, and the clinical phenotypic feature data of each of the pregnant women form a phenotypic feature vector of the pregnant woman;
(2) for each of the pregnant women, performing window division on at least part of a chromosome sequence of a reference genome to obtain sliding windows, counting the read segments falling within the sliding windows, and generating a sequence feature matrix of the chromosome sequence;
(3) for each of the pregnant women, constructing the training data set using the sequence feature matrix and the fetal chromosomal state, and training the machine learning model to extract a sequence feature vector of the chromosome sequence;
(4) combining the sequence feature vector and the phenotypic feature vector of each of the pregnant women to form a combined feature vector, and training the classification model with the combined feature vectors and the fetal chromosomal states of the pregnant women to obtain a trained classification detection model.

32. The method of Claim 31, the fetal chromosomal state of each of the pregnant women is one or more of: normal diploid, chromosomal aneuploid, partial monosomy syndrome, chromosomal microdeletion and chromosomal micro-duplication; preferably, the chromosomal aneuploidy includes at least one or more of: trisomy 21 syndrome, trisomy 18 syndrome and trisomy 13 syndrome; preferably, the partial monosomy syndrome includes 5p-syndrome.

33. The method of Claim 32 or 33, the number of the pregnant women is greater than 10, and the ratio of the number of fetuses with normal diploid to that of fetuses with chromosomal aneuploidy is ½ to 2.

34. The method of any one of Claims 31-33, in (3), the training data set is represented as:

$$D_{seq} = \{\mathbf{Z}_{i,j}^{(k)}, y^{(k)}\}_{k=1}^{N}$$

$$y^{(k)} = \begin{cases} 1, abnormal\ fetal\ chromosomes\ of\ sample\ k \\ 0, normal\ fetal\ chromosomes\ of\ sample\ k \end{cases}$$

wherein $N$ represents the number of training samples, and $N$ is an integer $\geq 1$; $\mathbf{Z}_{i,j}^{(k)}$ is the normalized sequence feature matrix of training sample $k$, and k$\in$[1,N], wherein i is an integer$\geq$ 1 and j is an integer$\geq$ 1; the chromosomal abnormality includes at least one or more of: trisomy 21 syndrome, trisomy 18 syndrome, trisomy 13 syndrome, 5p-syndrome, chromosomal microdeletion and chromosomal microduplication.

**35.** A system of detecting a fetal chromosomal abnormality, comprising:

a data acquisition module, for obtaining sequencing data of cell-free nucleic acid fragments and clinical phenotypic feature data from a pregnant woman sample to be detected, wherein the sequencing data comprise a plurality of read segments, and the clinical phenotypic feature data of the pregnant woman sample to be detected form a phenotypic feature vector of the pregnant woman;

a sequence feature matrix generation module, for performing window division on at least part of a chromosome sequence of a reference genome to obtain sliding windows, counting the read segments falling within the sliding windows, and generating a sequence feature matrix of the chromosome sequence;

a sequence feature vector extraction module, for inputting the sequence feature matrix into a trained machine learning model to extract a sequence feature vector of the chromosome sequence;

a classification detection module, for combining the sequence feature vector and the phenotypic feature vector of the pregnant woman to form a combined feature vector, and inputting the combined feature vector into a classification detection model to obtain the fetal chromosomal abnormality state of the pregnant woman to be detected.

**36.** The system of Claim 35, the system further includes an alignment module, for aligning the reads of the sequencing data to a reference genome to obtain the unique mapping reads.

**37.** The system of Claim 35 or 36, in the data acquisition module, the cell-free nucleic acid fragments are derived from the peripheral blood, liver, and/or placenta of the pregnant woman.

**38.** The system of any one of Claims 35-37, in the data acquisition module, the cell-free nucleic acid fragments are cell-free DNA.

**39.** The system of any one of Claims 35-38, in the data acquisition module, the sequencing data are derived from ultra-low depth sequencing; preferably, the sequencing depth of the ultra-low depth sequencing is $1\times$, $0.1\times$, or $0.01\times$.

**40.** The system of any one of Claims 35-39, in the data acquisition module, the read segments are aligned to the reference genome to obtain the unique mapping reads (preferably, GC content correction is performed); preferably, the subsequent steps are carried out with the unique mapping reads (preferably, the read segments are corrected by GC content).

**41.** The system of any one of Claims 35-40, in the data acquisition module, the phenotypic feature data of the pregnant woman are selected from one or combination of more of: age, gestational week, height, weight, BMI, biochemical test results of prenatal examination, ultrasonic diagnosis results, and cell-free fetal DNA concentration in plasma.

**42.** The system of any one of Claims 35-40, in the data acquisition module, the phenotypic feature data of the pregnant woman are subjected to outlier processing, missing value processing and/or null value processing.

**43.** The system of any one of Claims 35-42, in the data acquisition module, the phenotypic data of the pregnant woman sample will be judged as outliers if the following records appear:

a. $x_{age} < 10$ or $x_{age} > 80$;
b. $x_{GW} < 5$ or $x_{GW} > 50$;
c. $x_{height} < 40$ or $x_{height} > 300$;
d. $x_{weight} < 10$ or $x_{weight} > 200$;

and these outliers are set as a null value.

**44.** The system of Claim 42 or 43, the missing values and null values are padded by missForest algorithm.

**45.** The system of any one of Claims 35-44, in the sequence feature matrix generation module, the chromosome is chromosome 21, chromosome 18, chromosome 13 and/or a sex chromosome.

**46.** The system of any one of Claims 35-45, the following steps are performed in the sequence feature matrix generation module: (2.1) using the window with length b to overlap and slide the chromosome sequence with length L of the reference genome at step size of $t$ to obtain sliding windows, wherein b is a positive integer and b=[10000,10000000], t is any positive integer, L is a positive integer and L ≥ b; (2.2) counting the read segments falling within each of the sliding windows, and generating a sequence feature matrix of the chromosome sequence.

47. The system of any one of Claims 35-46, in the sequence feature matrix generation module, the sequence feature matrix includes the number, the base quality and the mapping quality of read segments within the sliding windows.

48. The system of Claim 47, the base quality includes the mean, the standard deviation, the skewness, and/or the kurtosis of the base quality.

49. The system of Claim 47, the mapping quality includes the mean, the standard deviation, the skewness and/or the kurtosis of the mapping quality.

50. The system of any one of Claims 35-49, in the sequence feature matrix generation module, the sequence feature matrix is:

$$X = \left( x_{ij} \right)_{h \times w}$$

wherein $h$ represents the number of sliding windows, w represents the number of sequence features within a single sliding window, and $x_{ij}$ represents the $j^{th}$ sequence eigenvalue in the $i^{th}$ sliding window.

51. The system of any one of Claims 35-50, in the sequence feature vector extraction module, the sequence feature matrix is normalized.

52. The system of any one of Claims 35-51, in the sequence feature vector extraction module, the sequence feature matrix is normalized by using formula (I):

$$Z_{i,j}^{(k)} = \frac{X_{i,j}^{(k)} - \mu_{i,j}}{\sigma_{i,j}}$$

$$(I)$$

wherein $Z_{i,j}^{(k)}$ is the normalized sequence feature matrix of sample $k$, $X_{i,j}^{(k)}$ represents the $j^{th}$ sequence eigenvalue in the $i^{th}$ sliding window of sample $k$, and $\mu_{i,j}$ and $\sigma_{i,j}$ represent the mean and standard deviation of the $j^{th}$ sequence eigenvalue in the $i^{th}$ sliding window of all samples, respectively.

53. The system of any one of Claims 35-52, in the sequence feature vector extraction module, the trained machine learning model is a neural network model or an AutoEncoder model; preferably, the neural network model is a deep neural network model, and more preferably, the neural network model is a deep neural network model based on 1D convolution.

54. The system of any one of Claims 35-53, in the classification detection module, the combined feature vector is obtained by combining the sequence feature vector and the phenotypic feature vector of the pregnant woman.

55. The system of any one of Claims 35-54, in the classification detection module, the combined feature vector $x$ is normalized by:

$$x_i' = \frac{x_i - \mu_i}{\sigma_i}$$

wherein $x_i'$ is the $i^{th}$ sequence eigenvalue of the normalized combined feature vector $x$, $x_i$ is the $i^{th}$ sequence eigenvalue of the combined feature vector $x$, $\mu_i$ is the mean of the $i^{th}$ sequence eigenvalues of the combined feature

vector *x*, and $\sigma_i$ is the standard deviation of the $i^{th}$ sequence eigenvalues of the combined feature vector.

56. The system of any one of Claims 35-55, in the classification detection module, the classification detection model is an ensemble learning model.

57. The system of Claim 56, the ensemble learning model is an ensemble learning model based on Stacking or Majority Voting; preferably, the ensemble learning model is one or more of: support vector machine model, naive Bayes classifier, random forest classifier, XGBoost and logistic regression.

58. A system of constructing a classification detection model for detecting a fetal chromosomal abnormality, comprising:

a data acquisition module, for obtaining sequencing data of cell-free nucleic acid fragments and clinical phenotypic feature data from of a pregnant woman, wherein the sequencing data comprise a plurality of read segments, and the fetal chromosomal state of the pregnant woman is known, and the clinical phenotypic feature data of the pregnant woman form a phenotypic feature vector of the pregnant woman;

a sequence feature matrix generation module, for performing window division on at least part of a chromosome sequence of a reference genome to obtain sliding windows, counting the read segments falling within the sliding windows, and generating a sequence feature matrix of the chromosome sequence;

a sequence feature vector extraction module, for constructing the training data set using the sequence feature matrix and the fetal chromosomal state, and training the machine learning model to extract a sequence feature vector of the chromosome sequence;

a classification detection module, for combining the sequence feature vector and the phenotypic feature vector of a pregnant woman to form a combined feature vector, and training the classification model with the combined feature vectors and the fetal chromosomal states of a plurality of pregnant women to obtain a trained classification detection model.

59. The system of Claim 58, the system further includes an alignment module, for aligning the read segments of the sequencing data to a reference genome to obtain the unique mapping reads.

FIG. 1

FIG. 2

FIG. 3

Input a feature map    $d \times h \times \text{f}$

Global average pooling    $d \times \text{f}$

Reshape    $d \times 1 \times \text{f}$

Fully connected layer (ReLU activation)    $d \times 1 \times \dfrac{\text{f}}{r_{SE}}$

Fully connected layer (sigmoid activation)    $d \times 1 \times \text{f}$

$\otimes$    $d \times h \times \text{f}$

FIG. 4

Input a feature map    $d \times h \times \text{f}$

1x1 1D convolution (sigmoid activation)    $d \times h \times 1$

$\otimes$    $d \times h \times \text{f}$

FIG. 5

Phenotypic data set
containing missing
values
(FF, Age, GW,
Height, Weight)

Training
set

Testing set

missing value
padding

missing value
padding

Padding model missForest

Calculate BMI

Calculate BMI

Phenotypic data set
after missing
value padding
(FF, Age, GW,
Height, Weight, BMI)

Training
set

Testing set

FIG. 6

A standardized complete feature vector in the training set

| SVC | $\nu$ -SVC | Gaussian NB | RF | XGB | Logistic Regression |

Base classifier
(primary classifier)

| P1 | P2 | P3 | P4 | P5 | P6 |

Prediction result of
the base classifier

ExtraTreesClassifier

Meta-classifier
(secondary classifier)

Output the probability of aneuploid

Final prediction result
of the meta-classifier

FIG. 7

ROC curve

FIG. 8

FIG. 9

FIG. 10

FIG. 11

Function with precision and recall as decision thresholds

FIG. 12

FIG. 13

## INTERNATIONAL SEARCH REPORT

| International application No. |
|---|
| **PCT/CN2020/132331** |

| | |
|---|---|
| **A.** | **CLASSIFICATION OF SUBJECT MATTER** |
| | G16B 20/10(2019.01)i |
| | According to International Patent Classification (IPC) or to both national classification and IPC |

| | |
|---|---|
| **B.** | **FIELDS SEARCHED** |
| | Minimum documentation searched (classification system followed by classification symbols) |
| | G16B |
| | Documentation searched other than minimum documentation to the extent that such documents are included in the fields searched |
| | |
| | Electronic data base consulted during the international search (name of data base and, where practicable, search terms used) |
| | CNKI, CNPAT, WPI, EPODOC: 染色体, 异常, 测序, 碱基质量, 比对质量, 游离DNA, 核酸片段, 孕妇, 机器学习, 卷积神经网络, 分类, 窗口, 胎儿, GC含量, 校正, 年龄, 体重, chromosome, sequencing, CNN, mapping, fetus, correct+, cf-DNA, pregnant, quality score |

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| Y | CN 111226281 A (SHENZHEN HUADA CLINIC EXAMINATION CENTER) 02 June 2020 (2020-06-02)<br>description paragraphs [0114]-[0174] | 1-59 |
| Y | CN 1600265 A (ZHENG, Mojing) 30 March 2005 (2005-03-30)<br>specific embodiments | 1-59 |
| Y | CN 111292802 A (ORIGIMED TECHNOLOGY (SHANGHAI) CO., LTD.) 16 June 2020 (2020-06-16)<br>description, paragraphs [0054]-[0080] | 1-59 |
| A | CN 111286529 A (CHANGZHOU MATERNAL AND CHILD HEALTH CARE HOSPITAL) 16 June 2020 (2020-06-16)<br>entire document | 1-59 |
| A | CN 111278993 A (THE REGENTS OF THE UNIVERSITY OF CALIFORNIA) 12 June 2020 (2020-06-12)<br>entire document | 1-59 |
| A | CN 111933213 A (NVIDIA CORP.) 13 November 2020 (2020-11-13)<br>entire document | 1-59 |

| ☑ Further documents are listed in the continuation of Box C. | ☑ See patent family annex. |
|---|---|

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **17 August 2021** | **30 August 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
| --- |
| **PCT/CN2020/132331** |

### C.    DOCUMENTS CONSIDERED TO BE RELEVANT

| Category* | Citation of document, with indication, where appropriate, of the relevant passages | Relevant to claim No. |
| --- | --- | --- |
| A | BENJAMINI, Y et al. "Summarizing and Correcting the GC Content Bias in High-throughput Sequencing" 《NUCLEIC ACIDS RESEARCH》 , Vol. 40, No. 10, 09 February 2012 (2012-02-09), ISSN: 0305-1048, page e72 | 6 |

Form PCT/ISA/210 (second sheet) (January 2015)

**INTERNATIONAL SEARCH REPORT**
**Information on patent family members**

International application No.
**PCT/CN2020/132331**

| Patent document cited in search report | Publication date (day/month/year) | Patent family member(s) | Publication date (day/month/year) |
|---|---|---|---|
| CN 111226281 A | 02 June 2020 | None | |
| CN 1600265 A | 30 March 2005 | None | |
| CN 111292802 A | 16 June 2020 | CN 111292802 B | 16 March 2021 |
| CN 111286529 A | 16 June 2020 | None | |
| CN 111278993 A | 12 June 2020 | EP 3682035 A1 | 22 July 2020 |
| | | US 2021125683 A1 | 29 April 2021 |
| | | WO 2019055835 A1 | 21 March 2019 |
| CN 111933213 A | 13 November 2020 | US 2020365234 A1 | 19 November 2020 |
| | | EP 3739590 A1 | 18 November 2020 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019191319 A1, Ehrich, Mathias

- WO 2020018522 A1, Egilsson, Agust

**Non-patent literature cited in the description**

- **EVANS, MARK I. ; STEPHANIE ANDRIOLE ; SHARA M. EVANS.** Genetics: update on prenatal screening and diagnosis. *Obstetrics and Gynecology Clinics,* 2015, vol. 42 (2), 193-208
- **NORWITZ, ERROL R ; BRYNN LEVY.** Noninvasive prenatal testing: the future is now. *Reviews in obstetrics and gynecology,* 2013, vol. 6 (2), 48
- **NORTON, MARY E et al.** Cell-free DNA analysis for noninvasive examination of trisomy. *New England Journal of Medicine,* 2015, vol. 372 (17), 1589-1597
- **LANGLOIS, SYLVIE et al.** Current status in non-invasive prenatal detection of Down syndrome, trisomy 18, and trisomy 13 using cell-free DNA in maternal plasma. *Journal of Obstetrics and Gynaecology Canada,* 2013, vol. 35 (2), 177-181
- **ALLYSE, MEGAN et al.** Non-invasive prenatal testing: a review of international implementation and challenges. *International journal of women's health,* 2015, vol. 7, 113
- **CHIU, ROSSA WK et al.** Noninvasive prenatal diagnosis of fetal chromosomal aneuploidy by massively parallel genomic sequencing of DNA in maternal plasma. *Proceedings of the National Academy of Sciences,* 2008, vol. 105 (51), 20458-20463
- **FAN, H. CHRISTINA et al.** Noninvasive diagnosis of fetal aneuploidy by shotgun sequencing DNA from maternal blood. *Proceedings of the National Academy of Sciences,* 2008, vol. 105 (42), 16266-16271
- **LAU, TZE KIN et al.** Noninvasive prenatal diagnosis of common fetal chromosomal aneuploidies by maternal plasma DNA sequencing. *The Journal of Maternal-Fetal & Neonatal Medicine,* 2012, vol. 25 (8), 1370-1374
- **JIANG, FUMAN et al.** Noninvasive Fetal Trisomy (NIFTY) test: an advanced noninvasive prenatal diagnosis methodology for fetal autosomal and sex chromosomal aneuploidies. *BMC medical genomics,* 2012, vol. 5 (1), 57

- **YANG, JIANFENG ; XIAOFAN DING ; WEIDONG ZHU.** Improving the calling of non-invasive prenatal testing on 13-/18-/21-trisomy by support vector machine discrimination. *BioRxiv,* 2017, 216689
- **XU, HANLI et al.** Informative priors on fetal fraction increase power of the noninvasive prenatal screen. *Genetics in Medicine,* 2018, vol. 20 (8), 817-824
- **PETERSEN, ANDREA K et al.** Positive predictive value estimates for cell-free noninvasive prenatal screening from data of a large referral genetic diagnostic laboratory. *American journal of obstetrics and gynecology,* 2017, vol. 217 (6), 691-e1
- **BENJAMINI, YUVAL ; TERENCE P. SPEED.** Summarizing and correcting the GC content bias in high-throughput sequencing. *Nucleic acids research,* 2012, vol. 40 (10), e72-e72
- **HU, JIE ; LI SHEN ; GANG SUN.** Squeeze-and-excitation networks. *Proceedings of the IEEE conference on computer vision and pattern recognition,* 2018
- Concurrent spatial and channel 'squeeze & excitation'in fully convolutional networks. **ROY, ABHIJIT GUHA ; NASSIR NAVAB ; CHRISTIAN WACHINGER.** International Conference on Medical Image Computing and Computer-Assisted Intervention. Springer, 2018
- **STEKHOVEN, DANIEL J ; PETER BÜHLMANN.** MissForest-non-parametric missing value imputation for mixed-type data. *Bioinformatics,* 2012, vol. 28 (1), 112-118
- Data Classification: Algorithms and Applications. **TANG, J. ; S. ALELYANI ; H. LIU.** Data Mining and Knowledge Discovery Series. CRC Press, 2015, 498-500
- **HE, KAIMING et al.** Delving deep into rectifiers: Surpassing human-level performance on imagenet classification. *Proceedings of the IEEE international conference on computer vision,* 2015